(19) Europäisches Patentamt
European Patent Office
Office européen des brevets

(11) EP 2 266 527 A2

(12) **EUROPÄISCHE PATENTANMELDUNG**

(43) Veröffentlichungstag:
**29.12.2010 Patentblatt 2010/52**

(21) Anmeldenummer: **10158297.1**

(22) Anmeldetag: **30.03.2010**

(51) Int Cl.:
***A61K 8/04*** *(2006.01)* ***A61K 8/25*** *(2006.01)*
***A61K 8/35*** *(2006.01)* ***A61K 8/49*** *(2006.01)*
***A61K 8/893*** *(2006.01)* ***A61K 8/91*** *(2006.01)*
***A61Q 17/04*** *(2006.01)*

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**
Benannte Erstreckungsstaaten:
**AL BA ME RS**

(30) Priorität: **23.06.2009 DE 102009027105**

(71) Anmelder: **Henkel AG & Co. KGaA**
**40589 Düsseldorf (DE)**

(72) Erfinder:
- **Janßen, Frank**
  **41470, Neuss (DE)**
- **Dickhof, Susanne**
  **41748, Viersen (DE)**
- **Yücel, Sevda**
  **41470, Neuss (DE)**
- **Waldmann-Laue, Marianne**
  **40789, Monheim (DE)**

(54) **Sonnenschutzzusammensetzungen mit verbessertem antioxidativen Potential**

(57) Gegenstand der vorliegenden Anmeldung sind kosmetische Zusammensetzungen mit verbessertem Lichtschutzfaktor, enthaltend mindestens ein Alkyl- und/oder Alkoxy-substituiertes Dibenzoylmethanderivat, Polysilicone-15, mindestens einen wasserlöslichen UV-Filter, umfassend ein Derivat der Benzimidazolsulfonsäure, sowie mindestens eine Substanz, ausgewählt aus 6,7-disubstituierten 2,2-Dialkylchromanen oder -chromenen der allgemeinen Formeln (I) oder (II),

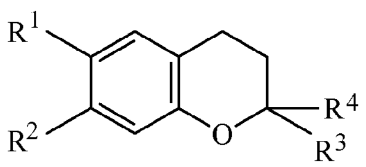

(I)

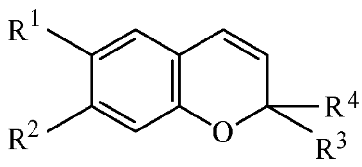

(II)

wobei $R^1$ und $R^2$ unabhängig voneinander eine OH-Gruppe, eine Methoxy-Gruppe oder eine $CF_3CH_2O$-Gruppe und $R^3$ und $R^4$ unabhängig voneinander eine $C_1$-$C_4$-Alkylgruppe darstellen, insbesondere Lipochroman-6.

EP 2 266 527 A2

## Beschreibung

Die vorliegende Erfindung betrifft kosmetische und dermatologische Zusammensetzungen zum Schutz von Haut und Haar gegen UV-Strahlung, die eine hohe antioxidative Wirkung aufweisen. Die Haut schädigende Wirkung des ultravioletten Teils der Sonnenstrahlung ist bekannt. Die UV-Strahlung im Bereich zwischen 290 nm und 320 nm, dem so genannten UV-B-Bereich, kann zu einem Erythem, einem einfachen Sonnenbrand oder sogar mehr oder weniger starken Verbrennungen als akuten Erscheinungsformen führen. Als ein Maximum der Erythemwirksamkeit des Sonnenlichtes wird der engere Bereich um 308 nm angegeben.

Man hat lange Zeit fälschlicherweise angenommen, dass die längerwellige UV-A-Strahlung mit einer Wellenlänge zwischen 320 nm und 400 nm nur eine vernachlässigbare biologische Wirkung aufweist. Inzwischen ist allerdings durch zahlreiche Studien belegt, dass UV-A-Strahlung im Hinblick auf die Auslösung photodynamischer, speziell phototoxischer Reaktionen und chronischer Veränderungen der Haut, weitaus gefährlicher als UV-B-Strahlung ist. Auch kann der schädigende Einfluss der UV-B-Strahlung durch UV-A-Strahlung noch verstärkt werden. So ist es u. a. erwiesen, dass selbst die UV-A-Strahlung unter ganz normalen Alltagsbedingungen ausreicht, um innerhalb kurzer Zeit die Collagen- und Elastinfasern zu schädigen, die für die Struktur und Festigkeit der Haut von wesentlicher Bedeutung sind. Hierdurch kommt es zu chronischen lichtbedingten Hautveränderungen - die Haut "altert" vorzeitig. Zum klinischen Erscheinungsbild der durch Licht gealterten Haut gehören beispielsweise Falten und Fältchen sowie ein unregelmäßiges, zerfurchtes Relief. Ferner können die von lichtbedingter Hautalterung betroffenen Partien eine unregelmäßige Pigmentierung aufweisen. Auch die Bildung von braunen Flecken, Keratosen und sogar Karzinomen bzw. malignen Melanomen ist möglich. Eine durch die alltägliche UV-Belastung vorzeitig gealterte Haut zeichnet sich außerdem durch eine geringere Aktivität der Langerhanszellen und eine leichte, chronische Entzündung aus.

Etwa 90 % der auf die Erde gelangenden ultravioletten Strahlung besteht aus UV-A-Strahlen. Während die UV-B-Strahlung in Abhängigkeit von zahlreichen Faktoren stark variiert (z. B. Jahres- und Tageszeit oder Breitengrad), bleibt die UV-A-Strahlung unabhängig von jahres- und tageszeitlichen oder geographischen Faktoren Tag für Tag relativ konstant. Gleichzeitig dringt der überwiegende Teil der UV-A-Strahlung in die lebende Epidermis ein, während etwa 70 % der UV-B-Strahlen von der Hornschicht zurückgehalten werden.

Es ist daher von grundsätzlicher Wichtigkeit, dass kosmetische und dermatologische Lichtschutzzusammensetzungen sowohl gegen UV-B- als auch gegen UV-A-Strahlung ausreichenden Schutz bieten. Hierzu sind im Stand der Technik schon eine Vielzahl von kosmetischen Zusammensetzungen entwickelt worden, die mindestens eine UV-Filtersubstanz enthalten.

Bei den UV-Filtersubstanzen handelt es sich um bei Raumtemperatur flüssig oder kristallin vorliegende Substanzen, die in der Lage sind, ultraviolette Strahlen zu absorbieren und die aufgenommene Energie in Form längerwelliger Strahlung, z. B. Wärme, wieder abzugeben. Man unterscheidet UV-A-Filter und UV-B-Filter, je nachdem, in welchem Wellenlängenbereich das Absorptionsmaximum des Filters liegt.

Die Zusammensetzungen des Standes der Technik sind weiter verbesserbar. Insbesondere weisen Zusammensetzungen, die Kombinationen von UV-Filtersubstanzen oder anderen Wirkstoffen enthalten, bisweilen in Bezug auf die Einsatzkonzentrationen formulierungstechnische Schwierigkeiten auf, die das Erreichen guter UV-Schutzleistung erschweren. Ein weiterer Nachteil des Standes der Technik ist, dass übliche Lichtschutzformulierungen einen meist klebrigen Film auf der Haut hinterlassen. Aufgabe der vorliegenden Erfindung war es, Lichtschutzzusammensetzungen bereitzustellen, die einen hohen Schutz gegen die oben genannten verschiedenen nachteiligen Wirkungen der Strahlung im UV-A-und UV-B-Bereich, insbesondere Sonnenstrahlung, bieten und gleichzeitig verbesserte sensorische Eigenschaften aufweisen. Die erfindungsgemäßen Zusammensetzungen sollen ferner gute hautpflegende Eigenschaften, insbesondere Wirksamkeit gegen Hautalterungserscheinungen (z.B. Faltenbildung, Elastizitätsverlust), sowie ein angenehmes Hautgefühl und eine leichte Verteilbarkeit auf der Haut aufweisen.

Die nicht vorveröffentlichte deutsche Patentanmeldung DE 10 2008 025 576 offenbart Sonnenschutzzusammensetzungen, die einen hohen Schutz gegen die oben genannten verschiedenen nachteiligen Wirkungen der Strahlung im UV-A-und UV-B-Bereich, insbesondere Sonnenstrahlung, bieten, verbesserte sensorische Eigenschaften aufweisen und gute hautpflegende Eigenschaften, insbesondere Wirksamkeit gegen Hautalterungserscheinungen (z.B. Faltenbildung, Elastizitätsverlust), sowie ein angenehmes Hautgefühl und eine leichte Verteilbarkeit auf der Haut aufweisen.

Es wurde nun gefunden, dass sich diese Eigenschaften auch durch eine Zusammensetzung erreichen lassen, die eine Kombination aus drei UV-Filtern und bestimmten Antioxidantien enthalten.

Gegenstand der vorliegenden Anmeldung sind kosmetische Zusammensetzung, enthaltend

a) mindestens einen öllöslichen UV-Filter, umfassend mindestens ein Alkyl- und/oder Alkoxy-substituiertes Dibenzoylmethanderivat,
b) Polysilicone-15,
c) mindestens einen wasserlöslichen UV-Filter, umfassend ein Derivat der Benzimidazolsulfonsäure,
d) mindestens eine Substanz, ausgewählt aus 6,7-disubstituierten 2,2-Dialkylchromanen oder -chromenen der allgemeinen Formeln (I) oder (II),

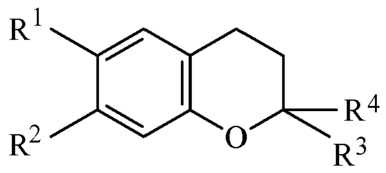

(I)

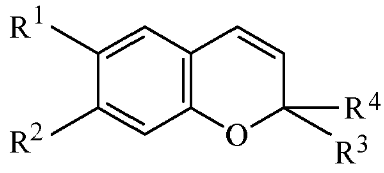

(II)

wobei $R^1$ und $R^2$ unabhängig voneinander eine OH-Gruppe, eine Methoxy-Gruppe oder eine $CF_3CH_2O$-Gruppe und $R^3$ und $R^4$ unabhängig voneinander eine $C_1$-$C_4$-Alkylgruppe darstellen, insbesondere Lipochroman-6,

wobei folgende Zusammensetzungen vom Schutzbereich ausgenommen sind:

| | | | | | | | | |
|---|---|---|---|---|---|---|---|---|
| Rizinusöl | - | - | - | - | - | - | - | 3,0 |
| C16-36 Alkyl Stearate | - | - | - | - | 1,0 | 2,0 | 1,0 | - |
| C20-40 Alkyl Stearate | - | - | - | - | 8,0 | 8, 0 | 9,0 | 8,0 |
| PEG-30 Stearat | 1,5 | - | - | - | - | - | - | - |
| PEG-40 Stearat | - | 2,0 | - | - | - | - | - | - |
| PEG-100 Stearat | - | - | - | 1,0 | - | - | - | - |
| Glyceryl Stearat | 0,5 | 1, 2 | - | 3,0 | - | - | - | - |
| Ceteareth-20 | 1,0 | - | - | - | - | - | - | - |
| Stearinsäure | 2,0 | - | - | - | - | - | - | - |
| Glyceryl Stearat Citrat | - | - | 3,5 | - | - | - | - | - |
| Cetyl Alkohol | 0,5 | 2,0 | 0,75 | 1,0 | - | - | - | - |
| Cetylpalmitat | - | 1,0 | - | - | - | - | - | 1,0 |
| Cetearylalkohol | - | - | - | - | - | - | 1,0 | - |
| Carnaubawachs | - | - | - | - | 1,5 | 0,5 | 2,0 | 2,0 |
| Veegum K = Mg-Al-Silikat | 0,8 | - | - | - | - | - | - | - |
| Xanthan Gummi | 0,2 | - | - | - | - | - | - | - |
| Carbomer | - | 0,3 | 0,2 | 0,2 | - | - | - | - |
| Hydroxyethylcellulose | 0,2 | - | - | - | - | - | - | - |
| Capryl-/Caprinsäure Triglycerid | - | - | 2,0 | - | 5,0 | 5,0 | 8,0 | 3,0 |
| Pentaerythrityl Tetraisostearat | - | - | - | - | 4,0 | - | 8,0 | - |
| Caprylylcarbonat | - | - | - | - | - | 5,0 | - | - |
| Jojobaöl | - | - | - | - | 1,0 | 1,0 | 1,0 | 1,0 |
| Lanolinöl | - | - | - | - | - | - | - | 1,0 |
| PEG-45/ Dodecyl Glycol Copolymer | - | - | - | - | 3,5 | 2,0 | 2,0 | 2,0 |
| Polyglyceryl-3 Diisostearat | - | - | - | - | - | 1,5 | 2,0 | 2,4 |
| Bis-Diglyceryl Polyacyladipat-2 | - | - | - | - | 2,0 | - | - | - |
| PVP / Eicosene Copolymer | - | - | - | - | - | - | 1,0 | 0,2 |
| Octyldodecanol | - | - | - | - | 5,0 | 5,0 | 8,0 | 6,0 |

(fortgesetzt)

| | | | | | | | | |
|---|---|---|---|---|---|---|---|---|
| Dicaprylylether | - | 3,0 | 2,0 | - | - | - | - | - |
| Dimethicone | 3,0 | 2,0 | - | - | - | - | - | - |
| Simethicone | - | - | - | - | 0,5 | 0,5 | - | 0,5 |
| Cyclomethicone | - | - | 4,0 | 1,0 | - | - | - | - |
| C12-C15 Alkyl Benzoat | 2,0 | - | - | - | - | - | - | - |
| Cetearyloctanoat | 2,0 | - | - | - | - | - | - | - |
| Squalan | 1,0 | - | - | 1,0 | - | - | - | - |
| Isopropylpalmitat | 1,0 | - | - | - | - | - | - | 2,0 |
| PPG-15 Stearylether | 2,0 | 2,0 | 3,0 | - | - | - | - | - |
| Vaseline | - | - | - | 2,0 | - | - | - | - |
| Hydrierte Kokosglyceride | 2,0 | - | - | 1,0 | - | - | - | - |
| Hydrierte Polydecen | | 2,0 | | | - | - | - | - |
| Stearyl Dimethicone | 9,0 | - | - | - | - | - | - | - |
| Ethylhexyl Methoxycinnamat | - | 4,50 | - | - | - | - | 1,0 | - |
| Bis-Ethylhexyloxyphenol methoxyphenyl Triazine | 1,00 | 2,50 | 3,00 | - | 0,50 | 1,50 | 3,50 | - |
| Butyl Methoxy Dibenzoylmethane | 2,00 | 2,50 | 3,00 | 2,00 | 3,0 | 3,0 | 3,0 | 2,00 |
| Neo Heliopan AP | - | - | - | 2,00 | - | - | - | 0,75 |
| Ethylhexyl Triazone | - | 1, 50 | - | - | 3,50 | - | - | - |
| Octocrylene | 3,00 | - | - | - | - | - | - | - |
| Diethylhexyl Butamido Triazone | - | - | 3,00 | - | - | 2,00 | 0,75 | |
| Neo Heliopan Hydro | 2,00 | 3,00 | 3,00 | - | - | - | - | - |
| Methylen Bis-Benzotriazolyl Tetramethylbutylphenol | 2,00 | 1,50 | - | - | - | - | - | 2,50 |
| Eusolex T-AVO | - | - | - | - | 2,0 | 1,0 | 4,0 | - |
| 2-Phenylbenzimidazol-5-sulfonsäure | - | - | - | - | 3,0 | 3,0 | 3,0 | 1,00 |
| Ethylhexyl Salicylate | - | - | - | - | - | 3,50 | - | - |
| Homosalate | - | - | - | - | 2,00 | - | - | - |
| Polysilicone-15 | 3,00 | 3,00 | 2,00 | 2,00 | 3,0 | 3,0 | 3,0 | 2,00 |
| Diethylhexyl-2,6-naphthalat | - | - | - | 3,50 | - | - | - | 5,50 |
| Ronasphere LDP | 1,0 | - | 4,0 | - | - | - | - | - |
| Tospearl 145A | - | 5, 0 | - | 3,0 | 3,0 | 0,5 | 1,0 | 5,0 |
| $BiOCl_3$ | - | - | - | - | - | 3,0 | - | - |
| Bornitrid | - | - | - | - | - | - | 3,0 | 1,0 |
| Lauroyl Lysin | - | - | - | - | 0,5 | - | - | - |
| Micropearl® M 310 | - | - | - | - | 6,0 | 3,0 | - | - |
| Siliciumdioxid-Partikel* | 2,00 | 1,50 | 2,00 | 1,50 | 2,00 | 2,00 | 2,00 | 2,00 |
| Eisenoxide CI 77491, 77492, 77499 | 1,2 | - | 0,8 | 2,6 | 3,6 | 1,8 | 0,8 | 3,2 |

(fortgesetzt)

| | | | | | | | | |
|---|---|---|---|---|---|---|---|---|
| Titandioxid, CI 77891 | 3,8 | - | 1,2 | 4,5 | 5,0 | 3, 6 | 1,2 | 4,5 |
| Ultramarin, CI 77007 | 0,5 | - | - | 0,6 | - | - | - | - |
| Interferenzpigmente | 0,8 | 6, 0 | - | - | - | - | 1,0 | - |
| D&C Rot 7 | - | - | - | - | - | - | - | 4,5 |
| D&C Rot 6 | - | - | - | - | - | - | - | 0,2 |
| FD&C Blau 1 | - | - | - | - | - | - | - | 0,5 |
| D&C Rot 21 | - | - | - | - | 0,1 | - | - | - |
| FD&C Gelb 6 | - | - | - | - | - | - | - | 0,2 |
| D&C Rot 34 | - | - | - | - | - | - | - | 0,5 |
| Glycerin | 2,0 | 2,0 | 5,0 | 10,0 | - | 10 | - | 10 |
| Citronensäure | - | - | - | - | - | 0,05 | - | - |
| Panthenol | - | - | - | - | - | - | 1,0 | - |
| Parfum | 1,0 | 1,0 | 1,0 | 1,0 | 1,0 | 1,0 | 1,0 | 1,0 |
| Methylparaben | 1,0 | 1,0 | 1,0 | 1,0 | 1,0 | 1,0 | 1,0 | 1,0 |
| Propylparaben | 1,0 | 1,0 | 1,0 | 1,0 | 1,0 | 1,0 | 1,0 | 1,0 |
| Lipochroman-6 | 0,5 | 0,5 | 0,5 | 0,5 | 0,5 | 0,5 | 0,5 | 0,5 |
| Wasser | ad 100 | ad 100 | ad 100 | ad 100 | ad 100 | ad 100 | ad 100 | ad 100 |
| * nicht organisch oder anorganisch modifiziert, keine organische oder anorganische Beschichtung, zu mehr als 80 Gew.-% sphärische Partikel, zahlenmittlerer Partikeldurchmesser 6 - 8 μm, Ölabsorptionskapazität : 0,9 - 1,3 $cm^3$ flüssiges Paraffin pro Gramm trockenes Siliciumdioxid (DIN 53601), erhalten durch Kieselsäure-Fällung | | | | | | | | |

und wobei folgende Zusammensetzungen ebenfalls vom Schutzbereich ausgenommen sind:

| | | | | |
|---|---|---|---|---|
| Emulsiphos 677660 | 3 | 3 | 3 | 3 |
| Cetiol B | 5 | 5 | 5 | 5 |
| Estasan GT8-60 3575 MCT Oil | 3,5 | 3,5 | 3,5 | 3,5 |
| Safloröl raffiniert | 2 | 2 | 2 | 2 |
| Behenylalkohol | 3 | 3 | 3 | 3 |
| Cetiol Sensoft | 1 | 1 | 1 | 1 |
| Vitamin E Acetat | 0,5 | 0,5 | 0,5 | 0,5 |
| Controx KS | 0,05 | 0,05 | 0,05 | 0,05 |
| Bienenwachs | 0,2 | 0,2 | 0,2 | 0,2 |
| COSMEDIA SP | 0,5 | 0,5 | 0,5 | 0,5 |
| Butylmethoxydibenzoylmethan | 3 | 3 | 3 | 3 |
| Parsol SLX | 3 | 3 | 3 | 3 |
| Propylparaben | 0,2 | 0,2 | 0,2 | 0,2 |
| Hexandiol-1,6 | 5 | 5 | 5 | 5 |
| Glycerin 86% pflanzlich | 4,5 | 4,5 | 4,5 | 4,5 |
| Sorbit 70% LS DAB | 2 | 2 | 2 | 2 |

(fortgesetzt)

| | | | | |
|---|---|---|---|---|
| Methylparaben | 0,2 | 0,2 | 0,2 | 0,2 |
| Tego Carbomer 140 | 0,5 | 0,5 | 0,5 | 0,5 |
| NTA-Na3 flüssig 40% | 0,1 | 0,1 | 0,1 | 0,1 |
| Cosmedia Silc | 2 | 2 | 2 | 2 |
| AMP Ultra PC 1000 | 0,99 | 0,99 | 0,99 | 0,99 |
| Neo Heliopan Hydro | 3 | 3 | 3 | 3 |
| Natriumhydroxid Perlen | 0,365 | 0,365 | 0,365 | 0,365 |
| Epicalmin TCM | - | - | 3 | 2 |
| Phycojuvenine | 2 | 1 | 2 | 1 |
| Lipochroman | 0,01 | 0,05 | 0,01 | 0,05 |
| Wasser, vollentsalzt | ad 100 | ad 100 | ad 100 | ad 100 |

Gegenstand der Erfindung ist ferner die Verwendung der erfindungsgemäßen vorstehend definierten Zusammensetzungen zum Schutz von Haut und Haar gegen die schädlichen Wirkungen von UV-Strahlung, insbesondere UV-A-und UV-B-Strahlung, insbesondere Sonnenstrahlung.

Bevorzugte Ausführungsformen der vorliegenden Erfindung ergeben sich aus den abhängigen Patentansprüchen.

Die erfindungsgemäßen Zusammensetzungen stellen in jeglicher Hinsicht überaus befriedigende Präparate dar, die im Hinblick auf die weiteren Bestandteile nicht auf eine spezielle Auswahl dieser weiteren Bestandteile begrenzt sind. Dementsprechend eignen sie sich ganz besonders, um als Grundlage für Zusammensetzungsformen mit vielfältigen Anwendungszwecken zu dienen. Die erfindungsgemäßen Zusammensetzungen zeigen überraschenderweise synergistisch verbesserte sensorische und kosmetische Eigenschaften, wie beispielsweise die gleichmäßige Verteilbarkeit auf der Haut oder das Einzugsvermögen in die Haut, und zeichnen sich gleichzeitig durch eine hohe Lichtschutzeffektivität bei gleichzeitig hervorragenden Hautpflegedaten aus.

Ein Maß für die UV-Schutzleistung stellt im Sinne der vorliegenden Erfindung beispielsweise der Lichtschutzfaktor (LSF bzw. SPF) dar.

Die kosmetischen und dermatologischen Zusammensetzungen im Sinne der vorliegenden Erfindung hinterlassen auf der Haut keinen schmierigen oder klebrigen Eindruck und sind ausgezeichnet hautverträglich.

a) Alkyl- und/oder Alkoxy-substituiertes Dibenzoylmethanderivat

Erfindungsgemäß bevorzugte Alkyl- und/oder Alkoxy-substituierte Dibenzoylmethanderivate sind **dadurch gekennzeichnet, dass** einer der Phenylreste mit mindestens einer Alkylgruppe, ausgewählt aus Methyl, Ethyl, n-Propyl, Isopropyl, n-Butyl, sec-Butyl, tert.-Butyl, n-Pentyl, Isopentyl, Neopentyl, 2-Ethylhexyl, und der andere Phenylrest mit mindestens einer Alkoxy-Gruppe, ausgewählt aus Methoxy, Ethoxy, n-Propoxy, Isopropoxy, n-Butoxy, sec-Butoxy, tert.Butoxy, n-Pentoxy, Isopentoxy, Neopentoxyl, 2-Ethylhexoxy, substituiert ist. Besonders bevorzugte Substituenten sind Isopropyl, tert.-Butyl und Methoxy. Erfindungsgemäß bevorzugte Alkyl- und/oder Alkoxy-substituierte Dibenzoylmethanderivate sind ausgewählt aus 2-Methyldibenzoylmethan, 4-Methyldibenzoylmethan, 4-Isopropyldibenzoylmethan, 4-tert-Butyldibenzoylmethan, 2,4-Dimethyldibenzoylmethan, 2,5-Dimethyldibenzoylmethan, 4,4'-Diisopropyldibenzoylmethan, 4,4'-Dimethoxydibenzoylmethan, 4-tert-Butyl-4'-Methoxydibenzoylmethan, 2-Methyl-5-isopropyl-4'-methoxydibenzoylmethan, 2-Methyl-5-tert-butyl-4'-methoxydibenzoylmethan, 2,4-Dimethyl-4'-methoxydibenzoylmethan, 2,6-Dimethyl-4-tert-butyl-4'-methoxydibenzoylmethan. Besonders bevorzugt ist 4-tert-Butyl-4'-Methoxydibenzoylmethan mit der INCI-Bezeichnung Butyl Methoxydibenzoylmethane (auch als 1-(4'-tert-Butylphenyl)-3-(4'-methoxyphenyl)propan-1,3-dion bezeichnet), ein öllöslicher organischer Lichtschutzfilter, der z. B. als PARSOL® 1789 von DSM oder als Eusolex® 9020 von Merck KGaA erhältlich ist.

b) Polysilicone-15

Die Substanz mit der INCI-Bezeichnung Polysilicone-15 wird auch als 3-(4-(2,2-Bis-Ethoxycarbonylvinyl)-phenoxy)propenyl)-methoxysiloxan/Dimethylsiloxan-Copolymer mit der Parsol® SLX), Dimethicodiethylbenzalmalonat,

Diethylbenzylidene Malonate Dimethicone oder Diethylmalonylbenzylidene Oxypropene Dimethicone bezeichnet und ist unter dem Handelsnamen Parsol® SLX (INCI-Bezeichnung Dimethicodiethylbenzal malonate (CAS-Nr. 207574-74-1)) von DSM erhältlich. Die chemische Struktur ist beispielsweise in EP 709080 A2 beschrieben.

c) Derivat der Benzimidazolsulfonsäure

Bevorzugte UV-Filtersubstanz(en) der Komponente (c) der erfindungsgemäßen Zusammensetzungen ist bzw. sind die Phenylen-1,4-bis-(2-benzimidazyl)-3,3'-5,5'-tetrasulfonsäure (UV-A) und ihre Salze, insbesondere die Alkali-, Erdalkali-, Ammonium-, Alkylammonium-, Alkanolammonium- und Glucammoniumsalze, bevorzugt die entsprechenden Natrium-, Kalium-, Trialkylammonium- oder Triethanolamin-Salze, insbesondere das Phenylen-1,4-bis-(2-benzimidazyl)-3,3'-5,5'-tetrasulfonsäure-bis-natriumsalz mit der INCI-Bezeichnung "Disodium Phenyl Dibenzimidazole Tetrasulfonate" (CAS-Nr.: 180898-37-7), das beispielsweise unter der Handelsbezeichnung Neo Heliopan AP von Symrise erhältlich ist. Weitere bevorzugte UV-Filtersubstanz(en) der Komponente (c) der erfindungsgemäßen Zusammensetzungen ist bzw. sind die 2-Phenylbenzimidazol-5-sulfonsäure (UV-B) und ihre Salze, insbesondere die Alkali-, Erdalkali-, Ammonium-, Alkylammonium-, Alkanolammonium- und Glucammoniumsalze, bevorzugt die entsprechenden Natrium-, Kalium-, Trialkylammonium- oder Triethanolamin-Salze, insbesondere aber die 2-Phenylbenzimidazol-5-sulfonsäure selbst mit der INCI-Bezeichnung "Phenylbenzimidazole sulfonic acid" (CAS.-Nr. 27503-81-7), die beispielsweise unter den Handelsnamen Neo Heliopan Hydro von Symrise oder Eusolex 232 von Merck KGaA erhältlich ist.

In einer erfindungsgemäß besonders bevorzugten Ausführungsform sind die Salze der 2-Phenylbenzimidazol-5-sulfonsäure ausgewählt aus den Salzen dieser Säure mit basischen Aminosäuren, insbesondere mit Lysin, Arginin und/oder Histidin, wobei Arginin besonders bevorzugt ist. In einer weiteren erfindungsgemäß besonders bevorzugten Ausführungsform sind die Salze der Phenylen-1,4-bis-(2-benzimidazyl)-3,3'-5,5'-tetrasulfonsäure ausgewählt aus den Salzen dieser Säure mit basischen Aminosäuren, insbesondere mit Ornithin, Lysin, Arginin und/oder Histidin, wobei Arginin besonders bevorzugt ist.

Erfindungsgemäß besonders bevorzugte Lichtschutzfilter-Kombinationen (a), (b) und (c) umfassen 4-tert-Butyl-4'-Methoxydibenzoylmethan, Polysilicone-15 und 2-Phenylbenzimidazol-5-sulfonsäure. Weitere erfindungsgemäß besonders bevorzugte Lichtschutzfilter-Kombinationen (a), (b) und (c) umfassen 4-tert-Butyl-4'-Methoxydibenzoylmethan, Polysilicone-15 und Dinatriumphenylbenzimidazoltetrasulfonat.

Weitere erfindungsgemäß besonders bevorzugte Lichtschutzfilter-Kombinationen (a), (b) und (c) umfassen 4-tert-Butyl-4'-Methoxydibenzoylmethan, Polysilicone-15 und ein 2-Phenylbenzimidazol-5-sulfonsäure-Arginin-Salz.

Die oben genannten UV-Filtersubstanzen der Komponente (a) sind bevorzugt in einer Gesamtmenge von 0,5 Gew.-% bis 20 Gew.-%, besonders bevorzugt 1 bis 15 Gew.-%, insbesondere 2 bis 10 Gew.-% und außerordentlich bevorzugt 3 - 5 Gew.-%, enthalten, jeweils bezogen auf das Gesamtgewicht der erfindungsgemäßen Zusammensetzungen.

Polysilicone-15, die oben genannte Komponente (b), ist bevorzugt in einer Gesamtmenge von 0,5 Gew.-% bis 10 Gew.-%, besonders bevorzugt 1 bis 7 Gew.-%, insbesondere 2 bis 5 Gew.-% und außerordentlich bevorzugt 3 - 4 Gew.-%, enthalten, jeweils bezogen auf das Gesamtgewicht der erfindungsgemäßen Zusammensetzungen.

Die oben genannten UV-Filtersubstanzen der Komponente (c) sind bevorzugt in einer Gesamtmenge von 0,5 Gew.-% bis 15 Gew.-%, besonders bevorzugt 1 bis 10 Gew.-%, insbesondere 2 bis 5 Gew.-% und außerordentlich bevorzugt 3 - 4 Gew.-%, enthalten, jeweils bezogen auf das Gesamtgewicht der erfindungsgemäßen Zusammensetzungen.

Bevorzugte erfindungsgemäße Zusammensetzungen sind **dadurch gekennzeichnet, dass** die UV-Filter a), b) und c) in einem Gewichtsverhältnis zueinander von a) : b) : c) wie (0,8 - 1,5) : (0,8 - 1,5) : (0,8 - 1,5), bevorzugt wie (0,9 - 1,2) : (0,9 - 1,2) : (0,9 - 1,2), besonders bevorzugt wie (1 - 1,1) : (1 - 1,1) : (1 - 1,1), enthalten sind.

Um den Lichtschutzfaktor weiter zu erhöhen, können die erfindungsgemäßen Zusammensetzungen bevorzugt mindestens eine weitere organische und/oder anorganische UV-Filtersubstanz enthalten. Weitere bevorzugte UV-Filtersubstanzen, die neben denen der Komponenten (a), (b) und (c) in den erfindungsgemäßen Zusammensetzungen eingesetzt werden, sind im Folgenden genannt.

Triazinderivate

UV-Filtersubstanzen auf Basis von Triazinderivaten, die das nachfolgende Strukturmotiv

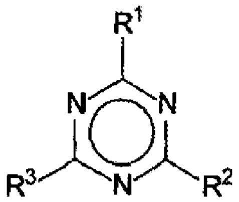

TRIAZIN-GRUND

aufweisen, sind im Stand der Technik bekannt, beispielsweise aus EP 775698, EP 878469 und EP 1027881.

Hinsichtlich der $C_3$-Achse des Triazin-Grundkörpers dieser Verbindungen sind sowohl symmetrische Substitution als auch unsymmetrische Substitution denkbar.

In diesem Sinne symmetrisch substituierte s-Triazine weisen drei gleiche Substituenten $R^1$, $R^2$ und $R^3$ auf, während unsymmetrisch substituierte s-Triazinderivate demzufolge unterschiedliche Substituenten aufweisen, wodurch die $C_3$-Symmetrie zerstört wird. Im Sinne der vorliegenden Erfindung wird als "unsymmetrisch" stets unsymmetrisch hinsichtlich der $C_3$-Achse des Triazin-grundkörpers verstanden, es sei denn, etwas anderes wäre ausdrücklich erwähnt.

Hinsichtlich der $C_3$-Achse des Triazin-Grundkörpers symmetrische Triazinderivate sind bevorzugt solche der allgemeinen Formel TRIAZIN-GRUND mit $R^1 = R^2 = R^3$ = -NH-Phe-COOR, mit Phe = Phenyl-Rest, bei dem die Substituenten -NH und -COOR in para-Position zueinander stehen. Besonders bevorzugte derartige symmetrische Triazinderivate sind 4,4',4"-(1,3,5-Triazin-2,4,6-triyltriimino)-tris-benzoesäure-tris(alkylester). Ein besonders bevorzugter derartiger Ester ist 4,4',4"-(1,3,5-Triazin-2,4,6-triyltriimino)-tris-benzoesäure-tris(2-ethylhexylester) [INCI: Ethylhexyl Triazone, vormals Octyl Triazone], das als Einzelsubstanz von BASF unter dem Handelsnamen UVINUL® T 150 vertrieben wird, aber auch in diversen kommerziellen UV-Filtermischungen erhältlich ist. Erfindungsgemäß bevorzugte unsymmetrische Triazinderivate sind beispielsweise solche, die in EP 775698 offenbart sind:

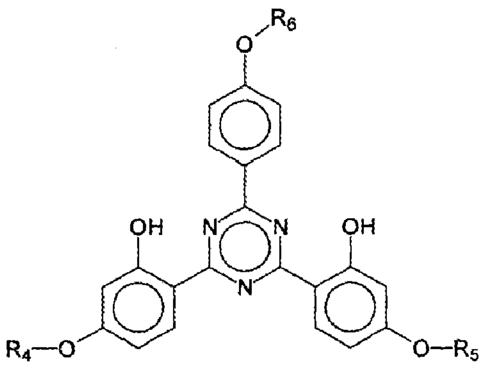

(BIS-RESOR-TRIAZIN)-I

und/oder

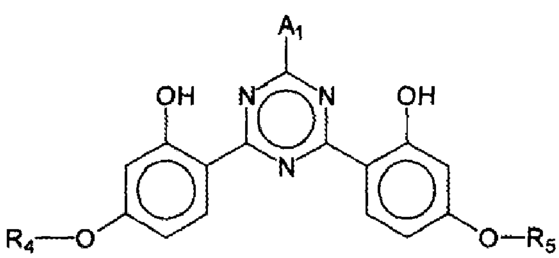

(BIS-RESOR-TRIAZIN)-II

Alle in EP 775698 erwähnten so genannten Bis-Resorcinyltriazine, seien sie durch generische oder durch

konkrete Formeln offenbart, sind vorteilhaft im Sinne der vorliegenden Erfindung. Ganz besonders bevorzugt werden $R_4$ und $R_5$ aus der Gruppe der verzweigten und unverzweigten Alkylgruppen von 1 bis 18 Kohlenstoffatomen gewählt. Auch können die Alkylgruppen wiederum vorteilhaft mit Silyloxygruppen substituiert sein.

$A_1$ stellt bevorzugt einen substituierten homo- oder heterocyclischen aromatischen Fünfring oder Sechsring dar.

Ganz besonders bevorzugte optionale UV-Filtersubstanzen sind ausgewählt aus unsymmetrisch substituierten s-Triazin-Verbindungen der allgemeinen Formel (BIS-RESOR-TRIAZIN)-I, in der $R_6$ ein Wasserstoffatom oder eine verzweigte oder unverzweigte Alkylgruppe mit 1 bis 10 Kohlenstoffatomen darstellt. Eine besonders bevorzugte Verbindung der allgemeinen Formel (BIS-RESOR-TRIAZIN)-I, in der $R_4$ und $R_5$ jeweils eine 2-Ethylhexyl-Gruppe und $R_6$ eine Methylgruppe darstellen, ist 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazine), unter dem Handelsnamen Tinosorb® S von CIBA erhältlich.

Eine weitere, erfindungsgemäß bevorzugte optionale unsymmetrisch substituierte s-TriazinVerbindung ist eine Verbindung mit der INCI-Bezeichnung Diethylhexyl Butamido Triazone, mit der allgemeinen Formel TRIAZIN-GRUND mit $R^1$ = $R^2$ -NH-Phe-COOR, mit Phe = Phenyl-Rest, bei dem die Substituenten -NH und -COOR in para-Position zueinander stehen, R = 2-Ethylhexyl und $R^3$ = -NH-Phe-CONH-tert-Butyl, mit Phe = Phenyl-Rest, bei dem die Substituenten -NH und -CONH-tert.-Butyl in para-Position zueinander stehen. Diese UV-Filtersubstanz, ein effektiver UV-A-Filter, ist unter der Handelsbezeichnung UVASORB HEB bei Sigma 3V erhältlich ist.

Weitere, erfindungsgemäß bevorzugte optionale UV-Filtersubstanzen auf Basis von s-Triazin-Verbindungen sind:

- 2,4,6-Tris([1,1'-Biphenyl]-4-yl)-1,3,5-Triazine, INCI: Tris-Biphenyl Triazine, erhältlich unter dem Handelsnamen Tinosorb A2B von CIBA,
- 2,4-bis-[5-1(di-methylpropyl)benzoxazol-2-yl-(4-phenyl)-imino]-6-(2-ethylhexyl)-imino-1,3,5-triazin (CAS Nr. 288254-16-0, Uvasorb® K2A von 3V Sigma, INCI: Ethylhexyl Bis-Isopentylbenzoxazolylphenyl Melamine),
- 2,4-Bis-{[4-(3-sulfonato)-2-hydroxy-propyloxy)-2-hydroxy]-phenyl}-6-(4-methoxyphenyl)-1,3,5-triazin-Natriumsalz,
- 2,4-Bis-{[4-(3-(2-propyloxy)-2-hydroxy-propyloxy)-2-hydroxy]-phenyl}-6-(4-methoxyphenyl)-1,3,5-triazin,
- 2,4-Bis-{[4-(2-ethyl-hexyloxy)-2-hydroxy]-phenyl}-6-[4-(2-methoxyethyl-carboxyl)-phenylamino]-1,3,5-triazin,
- 2,4-Bis-{[4-(3-(2-propyloxy)-2-hydroxy-propyloxy)-2-hydroxy]-phenyl}-6-[4-(ethylcarboxyl)-phenylamino]-1, 3,5-triazin,
- 2,4-Bis-{[4-(2-ethyl-hexyloxy)-2-hydroxy]-phenyl}-6-(1-methyl-pyrrol-2-yl)-1,3,5-triazin,
- 2,4-Bis-{[4-tris(trimethylsiloxy-silylpropyloxy)-2-hydroxy]-phenyl}-6-(4-methoxyphenyl)-1,3,5-triazin,
- 2,4-Bis-([4-(2-methylpropenyloxy)-2-hydroxy]-phenyl}-6-(4-methoxyphenyl)-1,3 ,5-triazin und
- 2,4-Bis-{[4-(1',1',1',3',5',5',5'-Heptamethylsiloxy-2-methyl-propyloxy)-2-hydroxy]-phenyl}-6-(4-methoxyphenyl)-1,3,5-triazin,
- 2-[4,6-Bis(2,4-dimethylphenyl)-1,3,5-triazin-2-yl]-5-(ethylhexyloxy)phenol.

Die s-Triazinderivate werden bevorzugt in die Ölphase der erfindungsgemäßen kosmetischen oder dermatologischen Zusammensetzungen eingearbeitet.

Benzotriazole

Eine weitere bevorzugte optionale UV-Filtersubstanz ist 2,2'-Methylen-bis-(6-(2H-benzotriazol-2-yl)-4-(1,1,3,3-tetramethylbutyl)-phenol) (INCI: Methylene Bis-Benzotriazolyl Tetramethylbutylphenol, MBBT) mit folgender Strukturformel

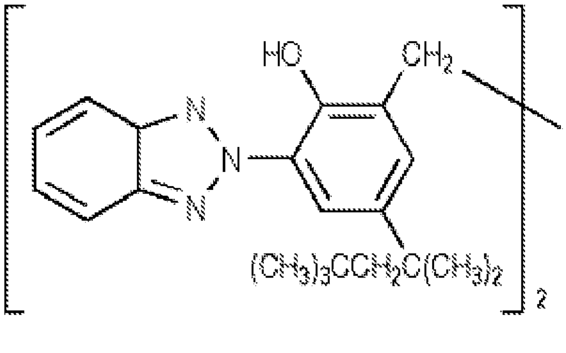

, unter der Handelsbezeichnung Tinosorb® M von CIBA erhältlich. Hierbei handelt es sich um einen so genannten

Breitbandfilter, der sowohl UV-A- als auch UV-B-Strahlung absorbiert.

Eine weitere bevorzugte optionale Breitband-UV-Filtersubstanz ist 2-(2H-Benzotriazol-2-yl)-4-methyl-6-[2-methyl-3-[1,3,3,3-tetramethyl-1-((trimethylsilyl)oxy]disiloxanyl]propyl]-phenol (CAS-Nr.: 155633-54-8) mit der INCI-Bezeichnung Drometrizole Trisiloxane.

Weitere bevorzugte optionale Benzotriazol-Filter sind 2,2'-Methyl-bis-[6(2H-benzotriazol-2-yl)-4-(methyl)phenol] (MIXXIM BB/200 der Firma Fairmount Chemical), 2-(2'-Hydroxy-3',5'-di-t-amylphenyl)benzotriazol (CAS- Nr.: 025973-551), 2-(2'-Hydroxy-5'-octylphenyl)-benzotriazol (CAS-Nr. 003147-75-9), 2-(2'-Hydroxy-5'-methylphenyl)benzotriazol (CAS-Nr. 2440-22-4) und 2-(2H-benzotriazol-2-yl)-4-methyl-6-[2-methyl-3-[1,3,3,3-tetramethyl-1-((trimethylsilyl)oxy]disiloxanyl)propyl]-phenol (CAS-Nr.: 155633-54-8) mit der INCI-Bezeichnung Drometrizole Trisiloxane.

Weitere bevorzugte UV-Filtersubstanzen, die neben denen der Komponenten (a), (b) und (c) in den erfindungsgemäßen Zusammensetzungen eingesetzt werden, sind im Folgenden genannt:

- Derivate des Camphers, insbesondere 3-Benzylidencampher-Derivate, die keine ionisierbaren funktionellen Gruppen im Molekül aufweisen können, bevorzugt 3-(4'-Methylbenzyliden)-D,L-campher [INCI: 4-Methylbenzylidene Camphor], das von Merck unter der Warenbezeichnung Eusolex 6300 vertrieben wird;
- Derivate des Camphers, die ionisierbare funktionelle Gruppen im Molekül aufweisen können, bevorzugt Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze; das 1,4-di(2-oxo-10-Sulfo-3-bornylidenmethyl)-Benzol und dessen Salze (besonders die entsprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird, Sulfonsäurederivate des 3-Benzylidencamphers, wie z. B. 4-(2-Oxo-3-bornylidenmethyl)benzolsulfonsäure und 2-Methyl-5-(2-oxo-3-bornyliden)-sulfonsäure und deren Salze mit der INCI-Bezeichnung Terephthalylidene Dicamphor Sulfonic Acid (CAS.-Nr.: 92761-26-7, als Mexoryl SX von der Firma Chimex erhältlich).
- 4-Aminobenzoësäure-Derivate, bevorzugt 4-(Dimethylamino)-benzoësäure(2-ethylhexyl)ester, 4-(Dimethylamino) benzoësäureamylester;
- 2-Aminobenzoësäure-Derivate
- Ester der Zimtsäure, bevorzugt 4-Methoxyzimtsäure(2-ethylhexyl)ester, 4-Methoxyzimtsäurepropylester, 4-Methoxyzimtsäureisopentylester; und 2-Cyano-3,3-phenylzimtsäure-2-ethylhexylester (= Ethylhexyl-2-cyano-3,3-diphenylacrylat (Octocrylene)),
- Ester der Salicylsäure, bevorzugt Salicylsäure(2-ethylhexyl)ester (2-Ethylhexylsalicylat (= Octylsalicylat)), Salicylsäure(4-isopropylbenzyl)ester (4-Isopropylbenzylsalicylat), Salicylsäurehomomenthylester (Homomenthylsalicylat, Homosalate).
- Derivate des Benzophenons, die keine ionisierbaren funktionellen Gruppen im Molekül aufweisen, bevorzugt 2-Hydroxy-4-methoxy-4'-methylbenzophenon, 2,2'-Dihydroxy-4-methoxybenzophenon, 2-Hydroxy-4-methoxybenzophenon, 2-Hydroxy-4-methoxy-4'-methylbenzophenon, 2,2'-Dihydroxy-4-methoxybenzophenon; 2-(4'-Diethylamino-2'-hydroxybenzoyl)-benzoesäurehexylester (auch: Aminobenzophenon, unter dem Namen Uvinul A Plus von BASF erhältlich),
- Derivate des Benzophenons, die ionisierbare funktionelle Gruppen im Molekül aufweisen, bevorzugt Sulfonsäurederivate von Benzophenonen, besonders bevorzugt 2-Hydroxy-4-methoxybenzophenon-5-sulfonsäure und ihre Salze,
- Ester der Benzalmalonsäure, bevorzugt 4-Methoxybenzalmalonsäuredi(2-ethylhexyl)ester,
- sowie an Polymere gebundene UV-Filter, die von Komponente (b) verschieden sind.
- Derivate von Benzoxazol, bevorzugt 2,2'(Naphthalene-1,4-Diyl)bis(benzoxazole) (INCI: Dibenzoxazoyl Naphthalene) und 2,4-bis-[5-1(di-methylpropyl)benzoxazol-2-yl-(4-phenyl)-imino]-6-(2-ethylhexyl)-imino-1,3,5-triazin (Uvasorb® K2A von 3V Sigma, INCI: Ethylhexyl Bis-Isopentylbenzoxazolylphenyl Melamine).

Die Benzoxazol-Derivate liegen bevorzugt in gelöster Form in den erfindungsgemäßen kosmetischen Zusammensetzungen vor. Es kann ggf. aber auch von Vorteil sein, wenn die Benzoxazol-Derivate in pigmentärer, d. h. ungelöster Form - beispielsweise in Partikelgrößen von 10 nm bis zu 300 nm - vorliegen.

Einige der öllöslichen UV-Filter können selbst als Lösungsmittel oder Lösungsvermittler für andere UV-Filter dienen. So lassen sich beispielsweise Lösungen des UV-A-Filters 1-(4-tert.-Butylphenyl)-3-(4'methoxyphenyl)propan-1,3-dion (z. B. Parsol® 1789) in verschiedenen UV-B-Filtern herstellen. Die erfindungsgemäßen Zusammensetzungen enthalten daher in einer weiteren bevorzugten Ausführungsform 1-(4-tert.-Butylphenyl)-3-(4'-methoxyphenyl)propan-1,3-dion in Kombination mit mindestens einem UV-B-Filter, ausgewählt aus 4-Methoxyzimtsäure-2-ethylhexylester, 2-Cyano-3,3-phenylzimtsäure-2-ethylhexylester, Salicylsäure-2-ethylhexylester und 3,3,5-Trimethyl-cyclohexylsalicylat. In diesen Kombinationen liegt das Gewichtsverhältnis von UV-B-Filter zu 1-(4-tert.-Butylphenyl)-3-(4'methoxyphenyl) propan-1,3-dion zwischen 1:1 und 10:1, bevorzugt zwischen 2:1 und 8:1, das molare Verhältnis liegt entsprechend zwischen 0,3 und 3,8, bevorzugt zwischen 0,7 und 3,0.

Die Liste der genannten optionalen UV-Filtersubstanzen, die im Sinne der vorliegenden Erfindung eingesetzt werden können, soll selbstverständlich nicht limitierend sein.

Die Gesamtmenge an der mindestens einen optionalen organischen UV-Filtersubstanz in den erfindungsgemäßen kosmetischen oder dermatologischen Zusammensetzungen beträgt bevorzugt 0,5 - 20 Gew.-%, besonders bevorzugt 1 - 15 Gew.-%, außerordentlich bevorzugt 2 - 10 Gew.-%, jeweils bezogen auf das Gesamtgewicht der erfindungsgemäßen Zusammensetzung. Bei den erfindungsgemäß bevorzugten optionalen anorganischen UV-Filtersubstanzen handelt es sich bevorzugt um feindisperse oder kolloiddisperse Metalloxide und Metallsalze, beispielsweise Titandioxid, Zinkoxid, Eisenoxid, Aluminiumoxid, Ceroxid, Zirkoniumoxid, Silicate (Talk) und Bariumsulfat. Besonders bevorzugt sind Titandioxid und Zinkoxid. Die Partikel sollten dabei einen mittleren Durchmesser von weniger als 100 nm, bevorzugt zwischen 5 und 50 nm und insbesondere zwischen 15 und 30 nm aufweisen, also so genannte Nanopigmente darstellen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z. B. Titandioxid T 805 (Degussa) oder Eusolex® T2000 (Merck). Als hydrophobe Coatingmittel kommen dabei vor allem Silicone und dabei speziell Trialkoxyoctylsilane, bevorzugt Triethoxy Caprylylsilane, oder Simethicone in Frage. Weitere bevorzugte Coatingmittel sind Aluminiumoxide. Weitere bevorzugte Coatingmittel ist Siliciumdioxid, z. B. bei dem Handelsprodukt Eusolex T AVO von Merck KGaA. Ein weiteres bevorzugtes Coatingmittel ist Stearinsäure. Ein weiteres bevorzugtes Coatingmittel ist Polyhydroxystearinsäure. Ein weiteres bevorzugtes Coatingmittel ist Aluminiumstearat. Ein besonders bevorzugter anorganischer UV-Filter ist ein mit Triethoxy Caprylylsilane hydrophob beschichtetes Titandioxid, erhältlich unter der Bezeichnung Titan M 265 von Kemira.

Erfindungsgemäß bevorzugt ist mindestens eine anorganische UV-Filtersubstanz in einer Gesamtmenge von 0,1 - 15 Gew.-%, besonders bevorzugt 0,5 - 10 Gew.-%, außerordentlich bevorzugt 1,0 - 5 Gew.-% und weiter bevorzugt 2,0 - 4,0 Gew.-%, jeweils bezogen auf die gesamte Zusammensetzung, enthalten.

Als Inhaltsstoff d) enthalten die erfindungsgemäßen Mittel 6,7-disubstituierte 2,2-Dialkylchromane oder -chromene der allgemeinen Formeln (I) oder (II),

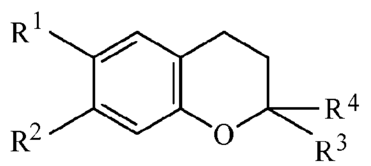

(I)

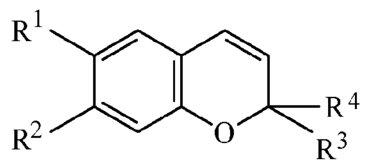

(II),

wobei $R^1$ und $R^2$ unabhängig voneinander eine OH-Gruppe, eine Methoxy-Gruppe oder eine $CF_3CH_2O$-Gruppe und $R^3$ und $R^4$ unabhängig voneinander eine $C_1$-$C_4$-Alkylgruppe darstellen.

Die Substituenten $R^1$ und $R^2$ sind unabhängig voneinander ausgewählt aus einer OH-Gruppe, einer Methoxy-Gruppe und einer $CF_3CH_2O$-Gruppe. In einer bevorzugten Ausführungsform sind $R^1$ und $R^2$ unabhängig voneinander ausgewählt aus einer OH-Gruppe und einer Methoxy-Gruppe. In einer besonders bevorzugten Ausführungsform stellen $R^1$ eine OH-Gruppe und $R^2$ eine Methoxy-Gruppe dar.

Die Substituenten $R^3$ und $R^4$ stellen unabhängig voneinander eine $C_1$-$C_4$-Alkylgruppe dar. Unter $C_1$-$C_4$-Alkylgruppe ist dabei erfindungsgemäß eine Methyl-, Ethyl-, n-Propyl-, Isopropyl-, n-Butyl-, Isobutyl- beziehungsweise 2-Methylpropyl-, sec-Butyl- beziehungsweise 1-Methylpropyl- oder eine tert.-Butyl-Gruppe zu verstehen. In einer bevorzugten Ausführungsform sind $R^3$ und $R^4$ unabhängig voneinander ausgewählt aus einer Methyl-, Ethyl-, n-Propyl-, Isopropyl- und einer n-Butyl-Gruppe. Besonders bevorzugt stellen $R^3$ und $R^4$ unabhängig voneinander eine Methyl- oder Ethylgruppe dar. Außerordentlich bevorzugt ist, dass $R^3$ und $R^4$ identisch sind.

Erfindungsgemäß bevorzugt verwendet werden die 6,7-disudstituierten 2,2-DialKylchromane. Ein erfindungsgemäß außerordentlich bevorzugt verwendeter Wirkstoff ist 2,2-Dimethyl-6-hydroxy-7-methoxy-chroman mit der systematischen Bezeichnung 3,4-Dihydro-7-methoxy-2,2-dimethyl-2H-1-benzopyran-6-ol und der INCI-Bezeichnung Dimethylmethoxy Chromanol. Die Substanz ist unter dem Handelsnamen Lipochroman-6 von der Firma Lipotec S.A. erhältlich.

Die 6,7-disubstituierten 2,2-Dialkylchromane oder -chromene der allgemeinen Formeln (I oder (II) werden vorzugsweise in Mengen von 0,001 - 5 Gew.-%, bevorzugt 0,005 - 2 Gew.-% und besonders bevorzugt 0,01 - 1 Gew.-%, jeweils bezogen auf die gesamte Zusammensetzung, eingesetzt.

Zusammenfassend sind erfindungsgemäße Zusammensetzungen bevorzugt, die - bezogen auf ihr Gewicht - 0,0001 bis 10 Gew.-%, vorzugsweise 0,0005 bis 7,5 Gew.-%, weiter bevorzugt 0,001 bis 5 Gew.-%, noch weiter bevorzugt 0,002 bis 2,5 Gew.-% und insbesondere 0,005 bis 2 Gew.-% 6,7-disubstituierte 2,2-Dialkylchromane oder -chromene

der allgemeinen Formeln (II) oder (III) wie vorstehend definiert, enthalten, wobei bevorzugte Mittel - bezogen auf ihr Gewicht - 0,0001 bis 10 Gew.-%, vorzugsweise 0,0005 bis 7,5 Gew.-%, weiter bevorzugt 0,001 bis 5 Gew.-%, noch weiter bevorzugt 0,002 bis 2,5 Gew.-% und insbesondere 0,005 bis 2 Gew.-% Lipochroman-6 enthalten.

Weitere erfindungsgemäß bevorzugte Zusammensetzungen enthalten - bezogen auf ihr Gewicht - eine Mischung aus der oxidierten und der reduzierten Form von 3,4-Dihydro-7-methoxy-2,2-dimethyl-2H-1-benzopyran-6-ol in einer Gesamtmenge von 0,001 - 2 Gew.-%, bevorzugt 0,005 - 1 Gew.-%, besonders bevorzugt 0,01 - 0,5 Gew.-%, außerordentlich bevorzugt 0,05 - 0,2 Gew.-%.

Ausdrücklich vom Schutzbereich ausgenommen sind folgende Zusammensetzungen:

| | 1 | 2 | 3 | 4 | 5 | 6 | 7 | 8 |
|---|---|---|---|---|---|---|---|---|
| Rizinusöl | - | - | - | - | - | - | - | 3,0 |
| C16-36 Alkyl Stearate | - | - | - | - | 1,0 | 2,0 | 1,0 | - |
| C20-40 Alkyl Stearate | - | - | - | - | 8,0 | 8, 0 | 9,0 | 8,0 |
| PEG-30 Stearat | 1,5 | - | - | - | - | - | - | - |
| PEG-40 Stearat | - | 2,0 | - | - | - | - | - | - |
| PEG-100 Stearat | - | - | - | 1,0 | - | - | - | - |
| Glyceryl Stearat | 0,5 | 1, 2 | - | 3,0 | - | - | - | - |
| Ceteareth-20 | 1,0 | - | - | - | - | - | - | - |
| Stearinsäure | 2,0 | - | - | - | - | - | - | - |
| Glyceryl Stearat Citrat | - | - | 3,5 | - | - | - | - | - |
| Cetyl Alkohol | 0,5 | 2,0 | 0,75 | 1,0 | - | - | - | - |
| Cetylpalmitat | - | 1,0 | - | - | - | - | - | 1,0 |
| Cetearylalkohol | - | - | - | - | - | - | 1,0 | - |
| Carnaubawachs | - | - | - | - | 1,5 | 0,5 | 2,0 | 2,0 |
| Veegum K = Mg-Al-Silikat | 0,8 | - | - | - | - | - | - | - |
| Xanthan Gummi | 0,2 | - | - | - | - | - | - | - |
| Carbomer | - | 0,3 | 0,2 | 0,2 | - | - | - | - |
| Hydroxyethylcellulose | 0,2 | - | - | - | - | - | - | - |
| Capryl-/Caprinsäure Triglycerid | - | - | 2,0 | - | 5,0 | 5,0 | 8,0 | 3,0 |
| Pentaerythrityl Tetraisostearat | - | - | - | - | 4,0 | - | 8,0 | - |
| Caprylylcarbonat | - | - | - | - | - | 5,0 | - | - |
| Jojobaöl | - | - | - | - | 1,0 | 1,0 | 1,0 | 1,0 |
| Lanolinöl | - | - | - | - | - | - | - | 1,0 |
| PEG-45/ Dodecyl Glycol Copolymer | - | - | - | - | 3,5 | 2,0 | 2,0 | 2,0 |
| Polyglyceryl-3 Diisostearat | - | - | - | - | - | 1,5 | 2,0 | 2,4 |
| Bis-Diglyceryl Polyacyladipat-2 | - | - | - | - | 2,0 | - | - | - |
| PVP / Eicosene Copolymer | - | - | - | - | - | - | 1,0 | 0,2 |
| Octyldodecanol | - | - | - | - | 5,0 | 5,0 | 8,0 | 6,0 |
| Dicaprylylether | - | 3,0 | 2,0 | - | - | - | - | - |
| Dimethicone | 3,0 | 2,0 | - | - | - | - | - | - |
| Simethicone | - | - | - | - | 0,5 | 0,5 | - | 0,5 |
| Cyclomethicone | - | - | 4,0 | 1,0 | - | - | - | - |

(fortgesetzt)

| | 1 | 2 | 3 | 4 | 5 | 6 | 7 | 8 |
|---|---|---|---|---|---|---|---|---|
| C12-C15 Alkyl Benzoat | 2,0 | - | - | - | - | - | - | - |
| Cetearyloctanoat | 2,0 | - | - | - | - | - | - | - |
| Squalan | 1,0 | - | - | 1,0 | - | - | - | - |
| Isopropylpalmitat | 1,0 - | | - | - | - | - | - | 2,0 |
| PPG-15 Stearylether | 2,0 | 2, 0 | 3,0 | - | - | - | - | - |
| Vaseline | - | - | - | 2,0 | - | - | - | - |
| Hydrierte Kokosglyceride | 2,0 | - | - | 1,0 | - | - | - | - |
| Hydrierte Polydecen | - | 2,0 | - | - | - | - | - | - |
| Stearyl Dimethicone | 9,0 | - | - | - | - | - | - | - |
| Ethylhexyl Methoxycinnamat | - | 4,50 | - | - | - | - | 1,0 | - |
| Bis-Ethylhexyloxyphenol methoxyphenyl Triazine | 1,00 | 2,50 | 3,00 | - | 0,50 | 1,50 | 3,50 | - |
| Butyl Methoxy Dibenzoylmethane | 2,00 | 2,50 | 3,00 | 2,00 | 3,0 | 3,0 | 3,0 | 2,00 |
| Neo Heliopan AP | - | - | - | 2,00 | - | - | - | 0,75 |
| Ethylhexyl Triazone | - | 1, 50 | - | - | 3,50 | - | - | - |
| Octocrylene | 3,00 | - | - | - | - | - | - | - |
| Diethylhexyl Butamido Triazone | - | - | 3,00 | - | - | 2,00 | 0,75 | |
| Neo Heliopan Hydro | 2,00 | 3,00 | 3,00 | - | - | - | - | - |
| Methylen Bis-Benzotriazolyl Tetramethylbutylphenol | 2,00 | 1,50 | - | - | - | - | - | 2,50 |
| Eusolex T-AVO | - | - | - | - | 2,0 | 1,0 | 4,0 | - |
| 2-Phenylbenzimidazol-5-sulfonsäure | - | - | - | - | 3,0 | 3,0 | 3,0 | 1,00 |
| Ethylhexyl Salicylate | - | - | - | - | - | 3,50 | - | - |
| Homosalate | - | - | - | - | 2,00 | - | - | - |
| Polysilicone-15 | 3,00 | 3,00 | 2,00 | 2,00 | 3,0 | 3,0 | 3,0 | 2,00 |
| Diethylhexyl-2,6-naphthalat | - | - | - | 3,50 | - | - | - | 5,50 |
| Ronasphere LDP | 1,0 | - | 4,0 | - | - | - | - | - |
| Tospearl 145A | - | 5, 0 | - | 3,0 | 3,0 | 0,5 | 1,0 | 5,0 |
| $BiOCl_3$ | - | - | - | - | - | 3,0 | - | - |
| Bornitrid | - | - | - | - | - | - | 3,0 | 1,0 |
| Lauroyl Lysin | - | - | - | - | 0,5 | - | - | - |
| Micropearl® M 310 | - | - | - | - | 6,0 | 3,0 | - | - |
| Siliciumdioxid-Partikel* | 2,00 | 1,50 | 2,00 | 1,50 | 2,00 | 2,00 | 2,00 | 2,00 |
| Eisenoxide CI 77491, 77492, 77499 | 1,2 | - | 0,8 | 2,6 | 3,6 | 1,8 | 0,8 | 3,2 |
| Titandioxid, CI 77891 | 3,8 | - | 1,2 | 4,5 | 5,0 | 3, 6 | 1,2 | 4,5 |
| Ultramarin, CI 77007 | 0,5 | - | - | 0,6 | - | - | - | - |
| Interferenzpigmente | 0,8 | 6, 0 | - | - | - | - | 1,0 | - |

(fortgesetzt)

| | 1 | 2 | 3 | 4 | 5 | 6 | 7 | 8 |
|---|---|---|---|---|---|---|---|---|
| D&C Rot 7 | - | - | - | - | - | - | - | 4,5 |
| D&C Rot 6 | - | - | - | - | - | - | - | 0,2 |
| FD&C Blau 1 | - | - | - | - | - | - | - | 0,5 |
| D&C Rot 21 | - | - | - | - | 0,1 | - | - | - |
| FD&C Gelb 6 | - | - | - | - | - | - | - | 0,2 |
| D&C Rot 34 | - | - | - | - | - | - | - | 0,5 |
| Glycerin | 2,0 | 2,0 | 5,0 | 10,0 | - | 10 | - | 10 |
| Citronensäure | - | - | - | - | - | 0,05 | - | - |
| Panthenol | - | - | - | - | - | - | 1,0 | - |
| Parfum | 1,0 | 1,0 | 1,0 | 1,0 | 1,0 | 1,0 | 1,0 | 1,0 |
| Methylparaben | 1,0 | 1,0 | 1,0 | 1,0 | 1,0 | 1,0 | 1,0 | 1,0 |
| Propylparaben | 1,0 | 1,0 | 1,0 | 1,0 | 1,0 | 1,0 | 1,0 | 1,0 |
| Lipochroman-6 | 0,5 | 0,5 | 0,5 | 0,5 | 0,5 | 0,5 | 0,5 | 0,5 |
| Wasser | ad 100 | ad 100 | ad 100 | ad 100 | ad 100 | ad 100 | ad 100 | ad 100 |
| * nicht organisch oder anorganisch modifiziert, keine organische oder anorganische Beschichtung, zu mehr als 80 Gew.-% sphärische Partikel, zahlenmittlerer Partikeldurchmesser 6 - 8 μm, Ölabsorptionskapazität : 0,9 - 1,3 $cm^3$ flüssiges Paraffin pro Gramm trockenes Siliciumdioxid (DIN 53601), erhalten durch Kieselsäure-Fällung | | | | | | | | |

Es handelt sich hierbei um Beispielrezepturen aus der nicht vorveröffentlichten deutschen Patentanmeldung DE 10 2008 025 576. Die Rezepturen 1 bis 4 sind wasserhaltige Stifte (1 = Abdeckstift; 2 = Foundation Stift; 3= Sonnenschutzstift; 4 = Lippenstift), die Beispielrezepturen 5 bis 8 sind O/W Emulsionen (5 = Foundation; 6 = Glanzcreme; 7 = Getönte Tagescreme; 8 = Foundation).

Liste der verwendeten Rohstoffe:

| Handelsname | INCI | Lieferant/Hersteller |
|---|---|---|
| Controx KS | Tocopherol, Hydrogenated Palm Glycerides Citrate | Cognis |
| Tego Carbomer 140 | Carbomer | Goldschmidt |
| Neo Heliopan AP | Disodium Phenyl Dibenzimidazol tetrasulfonate | Symrise |
| Neo Heliopan Hydro | Phenylbenzimidazol sulfonic acid | Symrise |
| Eusolex T-AVO | Titanium dioxide, Silica | Merck KGaA |
| Ronasphere LDP | Silica, CI 77891 (Titanium dioxide), CI 77491 (Iron Oxides) | Merck KGaA |
| Micropearl® M 310 | Methyl Methacrylate Crosspolymer | Seppic |
| Tospearl 145A | Polymethylsilsesquioxane | Momentive Performance Materials |
| Lipochroman-6 | Dimethylmethoxy Chromanol | Lipotec S.A. |

Bezüglich detaillierterer Angaben zu den eingesetzten Rohstoffen wird auf die ältere deutsche Patentanmeldung DE 10 2008 025 576 verwiesen.

Folgende Zusammensetzungen sind ebenfalls vom Schutzbereich ausgenommen:

| | 9 | 10 | 11 | 12 |
|---|---|---|---|---|
| Emulsiphos 677660 | 3 | 3 | 3 | 3 |
| Cetiol B | 5 | 5 | 5 | 5 |
| Estasan GT8-60 3575 MCT Oil | 3,5 | 3,5 | 3,5 | 3,5 |
| Safloröl raffiniert | 2 | 2 | 2 | 2 |
| Behenylalkohol | 3 | 3 | 3 | 3 |
| Cetiol Sensoft | 1 | 1 | 1 | 1 |
| Vitamin E Acetat | 0,5 | 0,5 | 0,5 | 0,5 |
| Controx KS | 0,05 | 0,05 | 0,05 | 0,05 |
| Bienenwachs | 0,2 | 0,2 | 0,2 | 0,2 |
| COSMEDIA SP | 0,5 | 0,5 | 0,5 | 0,5 |
| Butylmethoxydibenzoylmethan | 3 | 3 | 3 | 3 |
| Parsol SLX | 3 | 3 | 3 | 3 |
| Propylparaben | 0,2 | 0,2 | 0,2 | 0,2 |
| Hexandiol-1,6 | 5 | 5 | 5 | 5 |
| Glycerin 86% pflanzlich | 4,5 | 4,5 | 4,5 | 4,5 |
| Sorbit 70% LS DAB | 2 | 2 | 2 | 2 |
| Methylparaben | 0,2 | 0,2 | 0,2 | 0,2 |
| Tego Carbomer 140 | 0,5 | 0,5 | 0,5 | 0,5 |
| NTA-Na3 flüssig 40% | 0,1 | 0,1 | 0,1 | 0,1 |
| Cosmedia Silc | 2 | 2 | 2 | 2 |
| AMP Ultra PC 1000 | 0,99 | 0,99 | 0,99 | 0,99 |
| Neo Heliopan Hydro | 3 | 3 | 3 | 3 |
| Natriumhydroxid Perlen | 0,365 | 0,365 | 0,365 | 0,365 |
| Epicalmin TCM | - | - | 3 | 2 |
| Phycojuvenine | 2 | 1 | 2 | 1 |
| Lipochroman | 0,01 | 0,05 | 0,01 | 0,05 |
| Wasser, vollentsalzt | ad 100 | ad 100 | ad 100 | ad 100 |

Es handelt sich hierbei um Beispielrezepturen aus den nicht vorveröffentlichten deutschen Patentanmeldungen DE 10 2009 017 612 (9 und 10) und DE 10 2009 (11 und 12). Liste der verwendeten Rohstoffe:

| | |
|---|---|
| Emulsiphos 677660 | Potassium Cetyl Phosphate, Hydrogenated Palm Glycerides (Symrise) |
| Cetiol® B | Dibutyladipat (Cognis) |
| Estasan GT8-60 3575 | Caprylic acid, capric acid triglyceride (uniqema) |
| Cetiol Sensoft | Propylheptyl Caprylate (Cognis) |
| Cosmedia SP | Natriumpolyacrylat (Cognis) |
| Parsol® SLX | Dimethicodiethylbenzal malonat (INCI-Bezeichnung: Polysilicone-15, CAS-Nr.: 207574-74-1) (DSM Nutritional Products) |
| Cosmedia Silc | Silica (Cognis) |
| AMP Ultra PC 2000 | 2-Amino-2-methyl-propanol (ca. 94,5-95,4% Aktivsubstanz in Wasser; INCI-Bezeichnung: Aminomethyl Propanol) (Dow Chemical) |
| Neo Heliopan® Hydro | 2-Phenylbenzimidazol-2-sulfonsäure (INCI-Bezeichnung: Phenylbenzimidazole Sulfonic Acid) (Symrise) |

Bezüglich detaillierterer Angaben zu den eingesetzten Rohstoffen wird auf die beiden älteren deutschen Patentanmeldungen verwiesen.

Die erfindungsgemäßen kosmetischen und dermatologischen Zusammensetzungen können zusätzlich mindestens einen Feststoff enthalten, der aus Siliciumdioxid-Partikeln, die nicht organisch oder anorganisch modifiziert sind und keine organische oder anorganische Beschichtung aufweisen, ausgewählt ist. Bevorzugte Einsatzmengen hierfür liegen bei einer Gesamtmenge von 0,2 - 5 Gew.-%, bezogen auf die gesamte Zusammensetzung.

Das Merkmal "keine organische oder anorganische Beschichtung", bezieht sich erfindungsgemäß auf den Zustand der Siliciumdioxid-Partikel, bevor sie mit den übrigen Bestandteilen der erfindungsgemäßen Zusammensetzungen vermischt werden. Nach dem Vermischen ist es durchaus möglich, dass einzelne Rezepturbestandteile an der Oberfläche der Siliciumdioxid-Partikel adsorbiert oder absorbiert werden; das Merkmal "keine organische oder anorganische Beschichtung" bleibt davon unbenommen. Auch möglicherweise sorbierte Gase gelten erfindungsgemäß nicht als Beschichtung.

Eine erste erfindungsgemäß bevorzugte Ausführungsform ist **dadurch gekennzeichnet, dass** die Siliciumdioxid-Partikel durch Kieselsäure-Fällung erhältlich sind.

Eine weitere erfindungsgemäß bevorzugte Ausführungsform ist **dadurch gekennzeichnet, dass** die Siliciumdioxid-Partikel zu mehr als 80 Gew.-% sphärische Partikel umfassen.

Eine weitere erfindungsgemäß bevorzugte Ausführungsform ist **dadurch gekennzeichnet, dass** die Siliciumdioxid-Partikel einen zahlenmittleren Partikeldurchmesser im Bereich von 2 - 15 μm, besonders bevorzugt im Bereich von 5 - 10 μm, aufweisen.

Eine weitere erfindungsgemäß bevorzugte Ausführungsform ist **dadurch gekennzeichnet, dass** die Siliciumdioxid-Partikel eine Ölabsorptionskapazität von 0,7 - 1,5 $cm^3$ flüssiges Paraffin pro Gramm trockenes Siliciumdioxid, aufweisen, gemessen nach DIN 53601.

Eine weitere erfindungsgemäß bevorzugte Ausführungsform ist **dadurch gekennzeichnet, dass** die Siliciumdioxid-Partikel durch Kieselsäure-Fällung erhältlich sind und zu mehr als 80 Gew.-% sphärische Partikel umfassen.

Eine weitere erfindungsgemäß bevorzugte Ausführungsform ist **dadurch gekennzeichnet, dass** die Siliciumdioxid-Partikel durch Kieselsäure-Fällung erhältlich sind und einen zahlenmittleren Partikeldurchmesser im Bereich von 2 - 15 μm, besonders bevorzugt im Bereich von 5 - 10 μm, aufweisen.

Eine weitere erfindungsgemäß bevorzugte Ausführungsform ist **dadurch gekennzeichnet, dass** die Siliciumdioxid-Partikel durch Kieselsäure-Fällung erhältlich sind, zu mehr als 80 Gew.-% sphärische Partikel umfassen und einen zahlenmittleren Partikeldurchmesser im Bereich von 2 - 15 μm, besonders bevorzugt im Bereich von 5 - 10 μm, aufweisen.

Eine weitere erfindungsgemäß bevorzugte Ausführungsform ist **dadurch gekennzeichnet, dass** die Siliciumdioxid-Partikel durch Kieselsäure-Fällung erhältlich sind, zu mehr als 80 Gew.-% sphärische Partikel umfassen, einen zahlenmittleren Partikeldurchmesser im Bereich von 2 - 15 μm, besonders bevorzugt im Bereich von 5 - 10 μm, und eine Ölabsorptionskapazität von 0,7 - 1,5 $cm^3$ flüssiges Paraffin pro Gramm trockenes Siliciumdioxid, gemessen nach DIN 53601, aufweisen.

Bevorzugte Siliciumdioxid-Partikel sind als Handelsprodukt SB-705 der Firma Miyoshi Kasei, erhältlich, ein sphärisches Kieselgel mit der INCI-Bezeichnung Silica, das einen zahlenmittleren Teilchendurchmesser von 5 - 6 μm und eine spezifische Oberfläche von etwa 600 $m^2/g$ aufweist. Weitere bevorzugte Siliciumdioxid-Partikel sind als Handelsprodukt Aerosil von Evonik Degussa erhältlich: Aerosil 130, Aerosil 200, Aerosil 255, Aerosil 300 oder Aerosil 380. Weitere bevorzugte Siliciumdioxid-Partikel sind ebenfalls als Handelsprodukte erhältlich: CAB-O-SIL Fumed Silica (Cabot), CAB-O-SIL EH-5 (Cabot), CAB-O-SIL HS-5 (Cabot), CAB-O-SIL LM-130 (Cabot), CAB-O-SIL MS-55 (Cabot), CAB-O-SIL M-5 (Cabot), Cosmedia Silc (Cognis), Neosil PC 50 S (Ineos Silicas), Sorbosil BFG 54 (Ineos Silicas), Sorbosil AC33 (Ineos Silicas), Sorbosil AC 35 (Ineos Silicas), Sorbosil AC 37 (Ineos Silicas), Sorbosil AC 39 (Ineos Silicas), Wacker HDK H 30 (Wacker-Chemie), Wacker HDK N 20 (Wacker-Chemie), Wacker HDK S 13 (Wacker-Chemie), Wacker HDK T 30 (Wacker-Chemie), Wacker HDK V 15 (Wacker-Chemie).

Erfindungsgemäß bevorzugte Zusammensetzungen sind **dadurch gekennzeichnet, dass** sie die Siliciumdioxid-Partikel in einer Gesamtmenge von 0,2 - 5 Gew.-%, bevorzugt 1 - 4 Gew.-%, besonders bevorzugt 1,5 - 3,5 Gew.-%, außerordentlich bevorzugt 2 - 3 Gew.-% und weiter bevorzugt 2,5 - 2,8 Gew.-%, jeweils bezogen auf die gesamte Zusammensetzung, enthalten. Um die haptischen Eigenschaften und/oder den Lichtschutzfaktor der erfindungsgemäßen Zusammensetzungen weiter zu verbessern, können diese bevorzugt mindestens einen weiteren teilchenförmigen Feststoff, der von den Siliciumdioxid-Partikeln verschieden ist, enthalten. Weitere bevorzugte optionale teilchenförmige Feststoffe, die neben den Siliciumdioxid-Partikeln in den erfindungsgemäßen Zusammensetzungen eingesetzt werden, sind im Folgenden genannt. Bevorzugte optionale teilchenförmige Feststoffe sind ausgewählt aus färbenden, farbgebenden, mattierenden oder glanzgebenden Pigmenten. Bevorzugte Pigmente dieser Art können anorganisch oder organisch sein. Weitere bevorzugte Pigmente weisen einen zahlenmittleren Teilchendurchmesser von 0,1 - 200 μm, bevorzugt 0,5 - 100 μm, besonders bevorzugt 1 - 50 μm und außerordentlich bevorzugt 2 - 30 μm auf. Besonders bevorzugte anorganische Pigmente sind ausgewählt aus den Oxiden von Silicium, Titan, Eisen, Zink, Zirkonium, Magnesium, Cer und Bismut, aus Bismutoxychlorid, Bornitrid, Glimmer, Flussspat und wasserunlöslichen Perlglanzpigmenten, die mit mindestens einer anorganischen und/oder organischen Verbindung beschichtet sein können.

Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Besonders bevorzugte Farbpigmente sind beispielsweise Titandioxid, Glimmer, Eisenoxide (z. B. $Fe_2O_3$, $Fe_3O_4$ FeO(OH)) und/oder Zinnoxid. Besonders bevorzugte Farbstoffe sind beispielsweise Carmin, Berliner Blau, Chromoxidgrün, Ultramarinblau und/oder Manganviolett.

Erfindungsgemäß bevorzugte Perlglanzpigmente sind ausgewählt aus natürlichen Perlglanzpigmenten, wie z. B. "Fischsilber" (Guanin/Hypoxanthin-Mischkristalle aus Fischschuppen) und "Perlmutt" (vermahlene Muschelschalen), monokristallinen Perlglanzpigmenten, wie z. B. Bismuthoxychlorid (BiOCl), und Schicht-Substrat Pigmenten, z. B. Glimmer / Metalloxid. Basis für Perlglanzpigmente sind beispielsweise pulverförmige Pigmente oder Ricinusöldispersionen von Bismutoxychlorid und/oder Titandioxid sowie Bismutoxychlorid und/oder Titandioxid auf Glimmer. Insbesondere bevorzugt ist z. B. das unter der CIN 77163 aufgelistete Glanzpigment.

Erfindungsgemäß bevorzugt sind ferner die folgenden Perlglanzpigmentarten auf Basis von Metalloxid-beschichtetem Glimmer:

| Gruppe | Beschichtung / Schichtdicke | Farbe |
|---|---|---|
| Silberweiße Perlglanzpigmente $TiO_2$: | 40 - 60 nm | silber |
| Interferenzpigmente | $TiO_2$: 60 - 80 nm | gelb |
| | $TiO_2$: 80 - 100 nm | rot |
| | $TiO_2$: 100 - 140 nm | blau |
| | $TiO_2$: 120 - 160 nm | grün |
| Farbglanzpigmente | $Fe_2O_3$ | bronze |
| | $Fe_2O_3$ | kupfer |
| | $Fe_2O_3$ | rot |
| | $Fe_2O_3$ | rotviolett |
| | $Fe_2O_3$ | rotgrün |
| | $Fe_2O_3$ | schwarz |
| Kombinationspigmente | $TiO_2$/ $Fe_2O_3$ | Goldtöne |
| | $TiO_2$/ $Cr_2O_3$ | grün |
| | $TiO_2$/ Berliner Blau | tiefblau |
| | $TiO_2$/ Carmin | rot |

Erfindungsgemäß besonders bevorzugt sind z.B. die von der Firma Merck unter den Handelsnamen Timiron, Colorona oder Dichrona erhältlichen Perlglanzpigmente.

Die Liste der genannten Perlglanzpigmente soll selbstverständlich nicht limitierend sein. Im Sinne der vorliegenden Erfindung vorteilhafte Perlglanzpigmente sind auf zahlreichen, an sich bekannten Wegen erhältlich. Beispielsweise lassen sich außer Glimmer auch andere Substrate mit weiteren Metalloxiden beschichten, wie z. B. Silica und dergleichen mehr. Vorteilhaft sind z. B. mit $TiO_2$und $Fe_2O_3$ beschichtete $SiO_2$-Partikel ("Ronasphere LDP") von der Firma Merck, die sich besonders für die optische Reduktion feiner Fältchen eignen.

Es kann darüber hinaus erfindungsgemäß bevorzugt sein, gänzlich auf ein Substrat wie Glimmer zu verzichten. Besonders bevorzugt sind Perlglanzpigmente, die unter der Verwendung von $SiO_2$ hergestellt werden. Solche Pigmente, die auch zusätzlich goniochromatische Effekte haben können, sind z. B. unter dem Handelsnamen Sicopearl Fantastico bei der Firma BASF erhältlich. Weiterhin bevorzugt sind Pigmente der Firma Engelhard / Mearl auf Basis von Calcium Natrium Borosilikat, die mit Titandioxid beschichtet sind. Diese sind unter dem Namen Reflecks erhältlich. Sie weisen durch ihrer Partikelgröße von 40 - 180 $\mu$m zusätzlich zu der Farbe einen Glitzereffekt auf.

Besonders vorteilhaft sind ferner auch Effektpigmente, die unter der Handelsbezeichnung Metasomes Standard/Glitter in verschiedenen Farben (yellow, red, green, blue) von der Firma Flora Tech erhältlich sind. Die Glitterpartikel liegen hierbei in Gemischen mit verschiedenen Hilfs- und Farbstoffen (wie beispielsweise den Farbstoffen mit den Colour Index (CI) Nummern 19140, 77007, 77289, 77491) vor.

Die Farbstoffe und Pigmente können sowohl einzeln als auch im Gemisch vorliegen sowie gegenseitig miteinander beschichtet sein, wobei durch unterschiedliche Beschichtungsdicken im allgemeinen verschiedene Farbeffekte hervorgerufen werden.

Weitere bevorzugte Pigmente sind ausgewählt aus farbigen und farblosen Pigmenten. Einige der im Folgenden genannten Pigmente dienen auch als UV-Absorber. Besonders bevorzugte farbige Pigmente sind ausgewählt aus den Eisenoxiden mit den Color Index-Nummern CI 77491 (Eisenoxid rot), CI 77492 (Eisenoxidhydrat gelb) und CI 77499 (Eisenoxid schwarz), aus CI 77891 (Titandioxid) und Ruß.

Besonders bevorzugte Pigmente sind die Handelsprodukte SUNPMMA-S und SUNSIL Tin 30 der Firma Sunjin Chemicals Co. mit einem zahlenmittleren Teilchendurchmesser von 5 - 10 μm bzw. 2 - 7 μm.

Besonders bevorzugte optionale anorganische Pigmente sind beschichtet. Die Beschichtung kann mit Hilfe von anorganischen und/oder organischen Verbindungen erfolgen. Erfindungsgemäß besonders bevorzugt sind anorganische Pigmente, die eine anorganische Beschichtung aufweisen. Außerordentlich bevorzugte Pigmente dieser Art sind ausgewählt aus Siliciumdioxid-Partikeln, die mit Titandioxid und/oder Eisenoxiden beschichtet sind. Ein besonders bevorzugtes Pigment dieser Art ist das Handelsprodukt Ronasphere® LDP der Firma Merck KGaA. Bei diesem Produkt handelt es sich um sphärische Siliciumdioxid-Partikel, die mit Titandioxid und Eisenoxid beschichtet sind. Ronasphere® LDP weist einen zahlenmittleren Teilchendurchmesser von 4 - 7 μm auf. Weiterhin bevorzugt sind anorganisch beschichtete Glimmerpigmente, die keinen Perlglanz aufweisen. Weitere bevorzugte optionale anorganisch beschichtete anorganische Pigmente sind Glimmerpigmente, die mit Titandioxid in verschiedenen Schichtdicken beschichtet sind, beispielsweise die Produkte der Timiron®-Serie von Rona/Merck KGaA, insbesondere Pigmente der Produktreihen Timiron® MP, Timiron® Super, Timiron® Starlight und Timiron® Silk. Die genannten Produkte weisen mittlere Teilchendurchmesser von 5 - 60 μm bzw. 10 - 60 μm bzw. 10 - 125 μm bzw. 5 - 25 μm auf. Ebenfalls bevorzugt sind Glimmerpartikel mit einer Beschichtung aus Titandioxid und Eisenoxid, z. B. die Handelsprodukte Timiron® MP-20, MP-24, MP-25, MP-28, MP-29, MP-60 und MP-65. Weiterhin erfindungsgemäß bevorzugt sind mit Titandioxid und/oder rotem und/oder schwarzem Eisenoxid beschichtete Glimmerpartikel, z. B. die Produkte der Colorona®-Serie. Weitere bevorzugte Pigmente sind mit Kieselgel beschichtete Glimmerpigmente, z. B. das Handelsprodukt Micronasphere® M. Weitere erfindungsgemäß bevorzugte Pigmente sind anorganisch beschichtete anorganische Pigmente, deren Beschichtung einen Anteil von 0,1 - 1 Gew.% Zinnoxid aufweist.

Ebenfalls erfindungsgemäß bevorzugt sind anorganische Pigmente, die mit organischen Substanzen beschichtet sind. Bevorzugte Beispiele hierfür sind mit Aluminiumstearat beschichtete Titandioxid-Pigmente (z. B. das Handelsprodukt MT 100 T von der Firma Tayca), mit Dimethylpolysiloxan (Dimethicone) beschichtetes Zinkoxid, mit Dimethicone beschichtetes Bornitrid (Très BN® UHP 1106 von Carborundum), mit einem Gemisch aus Dimethylpolysiloxan und Silicagel (Simethicone) und Aluminiumoxidhydrat (Alumina) beschichtetes Titandioxid (Eusolex® T 2000 von Merck), mit Octylsilanol beschichtetes Titandioxid oder sphärische Polyalkylsesquisiloxan-Partikel (Aerosil® R972 von Degussa).

Weitere erfindungsgemäß besonders bevorzugte Zusammensetzungen sind dadurch gekennzeichnet, dass sie mindestens ein färbendes, farbgebendes, mattierendes oder glanzgebendes Pigment in einer Gesamtmenge von 0,1 Gew.-% bis 30 Gew.-%, bevorzugt 0,5 bis 15 Gew.-%, besonders bevorzugt 1,0 bis 10 Gew.-% und außerordentlich bevorzugt 2 - 5 Gew.-%, enthalten, jeweils bezogen auf das Gesamtgewicht der erfindungsgemäßen Zusammensetzung.

Weitere bevorzugte optionale teilchenförmige Feststoffe sind die durch Hydrolyse und Kondensationsreaktionen von Methyltrimethoxysilane erhältlichen Polymethylsilsesquioxane, die sphärisch sein können (insbesondere Aerosil® R 972 und Aerosil® 200 V von Evonik Degussa), und deren Teilchengrößenverteilung durch die Herstellung gesteuert werden kann.

Bevorzugte Polymethylsilsesquioxane werden beispielsweise unter den Handelsnamen Tospearl 2000 B und Tospearl 145A von Momentive Performance Materials, AEC Silicone Resin Spheres von A & E Connock sowie Wacker-Belsil PMS MK von der Wacker-Chemie angeboten.

Weitere bevorzugte optionale teilchenförmige Feststoffe sind ausgewählt aus gegebenenfalls modifizierten Stärken (z. B. aus Mais, Reis, Kartoffeln) und Stärkederivaten, die gewünschtenfalls vorverkleistert sind, insbesondere Stärkederivaten wie Natriumstärkeoctenylsuccinat, Aluminiumstärkeoctenylsuccinat oder Distarch Phosphate (z. B. Corn PO4 (Agrana Stärke), Corn PO4 (Tri-K)), Maisstärke Zea Mays (Amidon De Mais MST (Wackherr), Argo Brand Corn Starch (Corn Products), Pure-Dent (Grain Processing), Purity 21C (National Starch)), Cellulose, insbesondere mikrokristalliner Cellulose, und Cellulosederivaten, insbesondere Celluloseethern, wie Ethylcellulose, Hydroxyethylcellulose, Hydroxypropylcellulose oder Hydroxypropylmethylcellulose, Kieselgelen, Talkum, Kaolin, Silicaten, insbesondere Schichtsilicate und unter diesen insbesondere Montmorillonit, Kaolinit, Illit, Beidellit, Nontronit, Saponit, Hectorit, Bentoniten, Smectit, Magnesiumaluminiumsilikaten, insbesondere Talkum, Carbonaten, wie z. B. Magnesiumcarbonat ($MgCO_3$) und Calciumcarbonat ($CaCO_3$) .Bornitrid, Lactoglobulinderivaten, z. B. Natrium-$C_{8-16}$-Isoalkylsuccinyllactoglobulinsulfonat, von Brooks Industries erhältlich als Handelsprodukt Biopol® OE, Glaspulvern, Polymerpulvern, insbesondere aus Polyolefinen, Polycarbonaten, Polyurethanen, Polyamiden, z. B. Nylon, Polyestern, Polystyrolen, Polyacrylaten, (Meth)acrylat- oder (Meth)acrylat-Vinyliden-Copolymeren, die vernetzt sein können, oder Siliconen, sowie Mischungen dieser Substanzen. Bevorzugte Polymerpulver auf Basis eines Polymethacrylat-Copolymers sind z. B. als Handelsprodukt Polytrap® 6603 (Dow Corning) erhältlich. Andere bevorzugte Polymerpulver, z. B. auf Basis von Polyamiden, sind unter der Bezeichnung Orgasol® 1002 (Polyamid-6) und Orgasol® 2002 (Polyamid-12) von Elf Atochem erhältlich. Weitere Polymerpulver, die sich für den erfindungsgemäßen Zweck eignen, sind z. B. Polymethylmethacrylate (Micropearl® M von SEPPIC oder Plastic Powder A von NIKKOL), Styrol-Divinylbenzol-Copolymeren (Plastic Powder FP von NIKKOL), Polyethylen- und Polypropylen-Pulver (Microthene® von Omya oder ACCUREL® EP 400 von AKZO) oder auch Siliconpolymere (Silicone Powder X2-1605 von Dow Corning).

Die optionalen teilchenförmigen Feststoffe können sowohl einzeln als auch im Gemisch eingesetzt werden.

Weiterhin ist die Kombination der erfindungsgemäßen Siliciumdioxid-Partikel mit in Wasser Schichtsilikaten oder mit teilchenförmigen organischen Feststoffen bevorzugt.

Die Gesamtmenge der optionalen teilchenförmigen Feststoffe beträgt bevorzugt 0,1 - 30 Gew.-%, besonders bevorzugt 0,5 - 15 Gew.-%, außerordentlich bevorzugt 1,0 - 10 Gew.-%, oder auch 2 - 4 Gew.-%, jeweils bezogen auf das Gesamtgewicht der erfindungsgemäßen Zusammensetzung.

Die erfindungsgemäßen Zusammensetzungen können ferner kosmetische Hilfsstoffe und weitere Wirkstoffe enthalten, wie sie üblicherweise in solchen Zusammensetzungen verwendet werden, z. B. Konservierungsmittel, Konservierungshelfer, Bakterizide, Parfüms, Farbstoffe, Verdickungsmittel, anfeuchtende und/oder feuchthaltende Substanzen, Fette, Öle, Wachse, Alkohole, Polyole, Polymere, Elektrolyte, organische Lösemittel oder Siliconderivate.

Vorteilhafte Konservierungsmittel sind beispielsweise Formaldehydabspalter (wie z. B. DMDM Hydantoin), Iodopropylbutylcarbamate, Parabene, Phenoxyethanol, Ethanol, Benzoesäure und dergleichen mehr.

Bevorzugt umfasst das Konservierungssystem ferner auch Konservierungshelfer, wie beispielsweise Octoxyglycerin, 1,2-Pentandiol, 1,5-Pentandiol, 1,2-Hexandiol, 1,6-Hexandiol, 1,2-Heptandiol, 1,2-Octandiol, 1,8-Octandiol, 1,2-Nonandiol, 1,2-Decandiol, 1,10-Decandiol, 1,2-Undecandiol, 1,2-Dodecandiol, 1,2-Tridecandiol oder 1,2-Tetradecandiol.

Besonders bevorzugte Zusammensetzungen werden ferner erhalten, wenn als Zusatz- oder Wirkstoffe Antioxidantien eingesetzt werden. Erfindungsgemäß bevorzugt enthalten die Zusammensetzungen mindestens ein Antioxidans. Als günstige, aber dennoch fakultativ zu verwendende Antioxidantien können alle für kosmetische und/oder dermatologische Anwendungen geeigneten oder gebräuchlichen Antioxidantien verwendet werden, insbesondere Tocopherol, Tocopherylacetat oder Dimethylmethoxy Chromanol, erhältlich unter dem Handelsnamen Lipochroman-6 von Lipotec.

Weiter vorteilhaft werden der oder die Wirkstoffe gewählt aus der Gruppe, die Catechine und Gallensäureester von Catechinen und wässrige bzw. organische Extrakte aus Pflanzen oder Pflanzenteilen umfasst, die einen Gehalt an Catechinen oder Gallensäureestern von Catechinen aufweisen, wie beispielsweise den Blättern der Pflanzenfamilie Theaceae, insbesondere der Spezies Camellia sinensis (grüner Tee). Insbesondere vorteilhaft sind deren typische Inhaltsstoffe (wie z. B. Polyphenole bzw. Catechine, Coffein, Vitamine, Zucker, Mineralien, Aminosäuren, Lipide).

Catechine stellen eine Gruppe von Verbindungen dar, die als hydrierte Flavone oder Anthocyanidine aufzufassen sind und Derivate des "Catechins" (Catechol, 3,3',4',5,7-Flavanpentaol, 2-(3,4-Dihydroxyphenyl)-chroman-3,5,7-triol) darstellen. Auch Epicatechin ((2R, 3R)-3,3',4',5,7-Flavanpentaol) ist ein vorteilhafter Wirkstoff im Sinne der vorliegenden Erfindung.

Vorteilhaft sind ferner pflanzliche Auszüge mit einem Gehalt an Catechinen, insbesondere Extrakte des grünen Tees, wie z. B. Extrakte aus Blättern der Pflanzen der Spezies Camellia spec., ganz besonders der Teesorten Camellia sinenis, C. assamica, C. taliensis bzw. C. irrawadiensis und Kreuzungen aus diesen mit beispielsweise Camellia japonica. Bevorzugte Wirkstoffe sind ferner Polyphenole bzw. Catechine aus der Gruppe (-)-Catechin, (+)-Catechin, (-)-Catechingallat, (-)-Gallocatechingallat, (+)-Epicatechin, (-)-Epicatechin, (-)-Epicatechingallat, (-)-Epigallocatechin, (-)-Epigallocatechingallat. In einer weiteren bevorzugten Ausführungsform enthalten die erfindungsgemäßen Zusammensetzungen mindestens ein Flavonoid oder mindestens einen Flavonoid-reichen Pflanzenextrakt. Die erfindungsgemäß bevorzugten Flavonoide umfassen die Glycoside der Flavone, der Flavanone, der 3-Hydroxyflavone (Flavonole), der Aurone und der Isoflavone. Besonders bevorzugte Flavonoide sind ausgewählt aus Naringin (Aurantiin, Naringenin-7-rhamnoglucosid), $\alpha$-Glucosylrutin, $\alpha$-Glucosylmyricetin, $\alpha$-Glucosylisoquercetin, $\alpha$-Glucosylquercetin, Dihydroquercetin (Taxifolin), Hesperidin (3',5,7-Trihydroxy-4'-methoxyflavanon-7-rhamnoglucosid, Hesperitin-7-O-rhamnoglucosid), Neohesperidin, Rutin (3,3',4',5,7-Pentahydroxyflavon-3-rhamnoglucosid, Quercetin-3-rhamnoglucosid), Troxerutin (3,5-Dihydroxy-3',4',7-tris(2-hydroxyethoxy)-flavon-3-(6-O-(6-deoxy-$\alpha$-L-mannopyranosyl)-$\beta$-D-glucopyranosid)), Monoxerutin (3,3',4',5-Tetrahydroxy-7-(2-hydroxyethoxy)-flavon-3-(6-O-(6-deoxy-$\alpha$-L-mannopyranosyl)-$\beta$-D-glucopyranosid)), Diosmin (3',4',7-Trihydroxy-5-methoxyflavanon-7-rhamnoglucosid), Eriodictin und Apigenin-7-glucosid (4',5,7-Trihydroxyflavon-7-glucosid). Erfindungsgemäß außerordentlich bevorzugte Flavonoide sind $\alpha$-Glucosylrutin, Naringin und Apigenin-7-glucosid.

Ebenfalls bevorzugt sind die aus zwei Flavonoideinheiten aufgebauten Biflavonoide, die z. B. in Gingko-Arten vorkommen. Weitere bevorzugte Flavonoide sind die Chalkone, vor allem Phloricin, Hesperidinmethylchalkon und Neohesperidindihydrochalkon. Erfindungsgemäß besonders bevorzugte Zusammensetzungen sind **dadurch gekennzeichnet, dass** sie mindestens ein Flavonoid in einer Gesamtmenge von 0,0001 bis 1 Gew.-%, bevorzugt 0,0005 bis 0,5 Gew.-% und besonders bevorzugt 0,001 bis 0,1 Gew.-%, jeweils bezogen auf die Flavonoidaktivsubstanz in der

gesamten kosmetischen Zusammensetzung, enthalten.

In einer weiteren bevorzugten Ausführungsform enthalten die erfindungsgemäßen Zusammensetzungen mindestens ein Ubichinon oder ein Ubichinol oder deren Derivate. Ubichinole sind die reduzierte Form der Ubichinone. Die erfindungsgemäß bevorzugten Ubichinone weisen die Formel (UBI-I) auf:

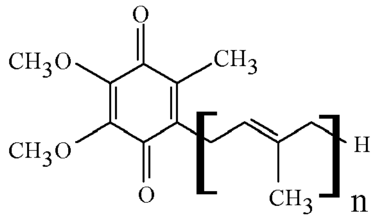

(UBI-I)

mit n = 6, 7, 8, 9 oder 10.

Besonders bevorzugt ist das Ubichinon der Formel (UBI-I) mit n = 10, auch bekannt als Coenzym Q10. Erfindungsgemäß besonders bevorzugte Zusammensetzungen sind dadurch gekennzeichnet, dass sie mindestens ein Ubichinon, Ubichinol oder ein Derivat hiervon in einer Gesamtmenge von 0,0001 - 1 Gew.-%, bevorzugt 0,001 - 0,5 Gew.-% und besonders bevorzugt 0,005 - 0,1 Gew.-%, jeweils bezogen auf die gesamte Zusammensetzung, enthalten.

In einer weiteren bevorzugten Ausführungsform enthalten die erfindungsgemäßen Zusammensetzungen Silymarin. Silymarin stellt erfindungsgemäß ein früher als einheitliche Substanz angesehenes Wirkstoff-Konzentrat aus den Früchten der Mariendistel (Silybum marianum) dar. Die Hauptbestandteile des Silymarins sind Silybin (Silymarin I), Silychristin (Silymarin II) und Silydianin, die zur Gruppe der Flavanolignane gehören. Erfindungsgemäß besonders bevorzugte Zusammensetzungen sind **dadurch gekennzeichnet, dass** sie Silymarin in Mengen von 0,00001 bis 1 Gew.-%, bevorzugt 0,0001 bis 0,01 Gew.-% und besonders bevorzugt 0,005 bis 0,1 Gew.-%, jeweils bezogen auf die gesamte Zusammensetzung, enthalten.

In einer weiteren bevorzugten Ausführungsform enthalten die erfindungsgemäßen Zusammensetzungen mindestens ein natürlich vorkommendes Xanthin-Derivat, ausgewählt aus Coffein, Theophyllin, Theobromin und Aminophyllin. Erfindungsgemäß bevorzugt sind die natürlich vorkommenden Xanthin-Derivate in Mengen von 0,0001 bis 1 Gew.-%, besonders bevorzugt 0,001 bis 0,5 Gew.-% und außerordentlich bevorzugt 0,005 bis 0,1 Gew.-%, jeweils bezogen auf die gesamte Zusammensetzung, enthalten.

In einer weiteren bevorzugten Ausführungsform enthalten die erfindungsgemäßen Zusammensetzungen Ectoin. Ectoin ist der Trivialname für 2-Methyl-1,4,5,6-tetrahydropyrimidin-4-carboxylat. Erfindungsgemäß bevorzugt ist Ectoin in Mengen von 0,0001 bis 1 Gew.-%, besonders bevorzugt 0,001 bis 0,5 Gew.-% und außerordentlich bevorzugt 0,005 bis 0,01 Gew.-%, jeweils bezogen auf die gesamte Zusammensetzung, enthalten.

In einer weiteren bevorzugten Ausführungsform enthalten die erfindungsgemäßen Zusammensetzungen mindestens eine natürliche Betainverbindung. Erfindungsgemäß bevorzugte natürliche Betainverbindungen sind natürlich vorkommende Verbindungen mit der Atomgruppierung $R_3N^+$-$CH_2$-X-$COO^-$ gemäß IUPAC-Regel C-816.1. Sogenannte Betaintenside (synthetisch) fallen nicht unter die erfindungsgemäß verwendeten Betainverbindungen, ebenso wenig andere zwitterionische Verbindungen, in denen sich die positive Ladung an N oder P und die negative Ladung formal an O, S, B oder C befindet, die aber nicht der IUPAC-Regel C-816.1 entsprechen. Erfindungsgemäß bevorzugte Betainverbindungen sind Betain ($Me_3N^+$-$CH_2$-$COO^-$), Carnitin ($Me_3N^+$-$CH_2$-CHOH-$CH_2$-$COO^-$), jeweils mit Me = Methyl, und Ergothionein = 2-Mercapto-$N^{\alpha}N^{\alpha}N^{\alpha}$-trimethyl-L-histidinium-Betain der Formel (BETA-II)

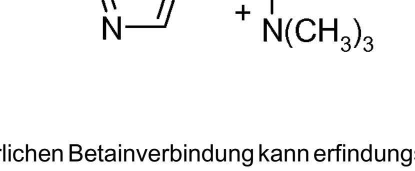

(BETA-II).

Die Herkunft der eingesetzten natürlichen Betainverbindung kann erfindungsgemäß durchaus synthetisch sein. Weiterhin

können auch Salze und/oder Ester der natürlichen Betainverbindungen erfindungsgemäß bevorzugt eingesetzt werden. Besonders bevorzugte derartige Derivate sind Carnitintartrat, Propionylcarnitin und insbesondere Acetylcarnitin. Beide Enantiomere (D-und L-Form) sind vorteilhaft im Sinne der vorliegenden Erfindung. Es kann auch von Vorteil sein, beliebige Enantiomerengemische, beispielsweise ein Racemat aus D-und L-Form, zu verwenden. Bevorzugte erfindungsgemäße Zusammensetzungen sind **dadurch gekennzeichnet, dass** sie mindestens eine natürliche Betainverbindung in einer Gesamtmenge von 0,01 bis 5 Gew.-%, bevorzugt 0,1 bis 3 Gew.-%, besonders bevorzugt 0,2 bis 1 Gew.-% und außerordentlich bevorzugt 0,3 - 0,5 Gew.-%, jeweils bezogen auf die gesamte erfindungsgemäße Zusammensetzung, enthalten.

Weitere erfindungsgemäß bevorzugte Zusammensetzungen sind dadurch gekennzeichnet, dass sie mindestens einen konditionierenden Wirkstoff enthalten. Unter konditionierenden Wirkstoffen sind erfindungsgemäß solche Substanzen zu verstehen, die auf keratinische Materialien, insbesondere auf die Haut, aufziehen und die physikalischen und sensorischen Eigenschaften sowohl der Haut als auch des Produktes als solchem verbessern. Konditionierungsmittel glätten die oberste Schicht der Haut und machen sie weich und geschmeidig. Über die Auswahl der Konditionierungsmittel (fettig - weniger fettig, schnell oder langsam spreitend, schnell oder langsam in die Haut einziehend und so weiter) lässt sich das Hautgefühl des gesamten Produktes einstellen. Erfindungsgemäß bevorzugte konditionierende Wirkstoffe sind ausgewählt aus Fettstoffen, insbesondere pflanzlichen Ölen, wie Sonnenblumenöl, Olivenöl, Sojaöl, Rapsöl, Mandelöl, Jojobaöl, Orangenöl, Weizenkeimöl, Pfirsichkernöl und den flüssigen Anteilen des Kokosöls, Lanolin und seinen Derivaten, flüssigen Paraffinölen, Isoparaffinölen und synthetischen Kohlenwasserstoffen, Di-n-alkylethern mit insgesamt 12 bis 36 C-Atomen, z. B. Di-n-octylether und n-Hexyl-n-octylether, Fettsäuren, besonders linearen und/oder verzweigten, gesättigten und/oder ungesättigten $C_{8-30}$-Fettsäuren, Fettalkoholen, besonders gesättigten, ein- oder mehrfach ungesättigten, verzweigten oder unverzweigten Fettalkoholen mit 4 - 30 Kohlenstoffatomen, die mit 1 - 75, bevorzugt 5 - 20 Ethylenoxid-Einheiten ethoxyliert und/oder mit 3 - 30, bevorzugt 9 - 14 Propylenoxid-Einheiten propoxyliert sein können, Esterölen, das heißt Estern von $C_{6-30}$-Fettsäuren mit $C_{2-30}$-Fettalkoholen, Hydroxycarbonsäurealkylestern, Dicarbonsäureestern wie Di-n-butyladipat sowie Diolestern wie Ethylenglykoldioleat oder Propylenglykoldi(2-ethylhexanoat), symmetrischen, unsymmetrischen oder cyclischen Estern der Kohlensäure mit Fettalkoholen, z. B. Glycerincarbonat oder Dicaprylylcarbonat (Cetiol® CC), Mono,- Di- und Trifettsäureestern von gesättigten und/oder ungesättigten linearen und/oder verzweigten Fettsäuren mit Glycerin, die mit 1 - 10, bevorzugt 7 - 9 Ethylenoxid-Einheiten ethoxyliert sein können, z. B. PEG-7 Glyceryl Cocoate, Wachsen, insbesondere Insektenwachsen, Pflanzenwachsen, Fruchtwachsen, Ozokerit, Mikrowachsen, Ceresin, Paraffinwachsen, Triglyceriden gesättigter und gegebenenfalls hydroxylierter $C_{16-30}$-Fettsäuren, z. B. gehärteten Triglyceridfetten, Phospholipiden, beispielsweise Sojalecithin, EiLecithin und Kephalinen, Siliconverbindungen, ausgewählt aus Decamethylcyclopentasiloxan, Dodecamethylcyclohexasiloxan und Siliconpolymeren, die gewünschtenfalls quervernetzt sein können, z. B. Polydialkylsiloxanen, Polyalkylarylsiloxanen, ethoxylierten und/oder propoxylierten Polydialkylsiloxanen mit der früheren INCI-Bezeichnung Dimethicone Copolyol, sowie Polydialkylsiloxanen, die Amin- und/oder Hydroxy-Gruppen enthalten, bevorzugt Substanzen mit den INCI-Bezeichnungen Dimethiconol, Amodimethicone oder Trimethylsilylamodimethicone.

Die Einsatzmenge der Fettstoffe beträgt bevorzugt 0,1 - 99 Gew.-%, besonders bevorzugt 2 - 50 Gew.-% und außerordentlich bevorzugt 5 - 20 Gew.-%, jeweils bezogen auf die gesamte erfindungsgemäße Zusammensetzung.

Bevorzugt liegen die erfindungsgemäßen kosmetischen oder dermatologischen Zusammensetzungen in Form einer flüssigen, fließfähigen oder festen Öl-in-Wasser-Emulsion, Wasser-in-Öl-Emulsion, Mehrfach-Emulsion, insbesondere einer Öl-in-Wasser-in-Öl- oder Wasser-in-Öl-in-Wasser-Emulsion, Makroemulsion, Miniemulsion, Mikroemulsion, PIT-Emulsion, Nanoemulsion, Pickering-Emulsion, Hydrodispersion, eines Hydrogels, eines Lipogels, einer ein- oder mehrphasigen Lösung, eines Schaumes, eines Puders oder einer Mischung mit mindestens einem als medizinischen Klebstoff geeigneten Polymer vor. Die Mittel können auch in wasserfreier Form, wie beispielsweise einem Öl oder einem Balsam, dargereicht werden. Hierbei kann der Träger ein pflanzliches oder tierisches Öl, ein Mineralöl, ein synthetisches Öl oder eine Mischung solcher Öle sein. In einer besonders bevorzugten Ausführungsform der erfindungsgemäßen Mittel liegen die Mittel als Mikroemulsion vor. Unter Mikroemulsionen werden im Rahmen der Erfindung neben den thermodynamisch stabilen Mikroemulsionen auch die so genannten "PIT"-Emulsionen verstanden. Bei diesen Emulsionen handelt es sich um Systeme mit den 3 Komponenten Wasser, Öl und Emulgator, die bei Raumtemperatur als Öl-in-Wasser-Emulsion vorliegen. Beim Erwärmen dieser Systeme bilden sich in einem bestimmten Temperaturbereich (als Phaseninversionstemperatur oder "PIT" bezeichnet) Mikroemulsionen aus, die sich bei weiterer Erwärmung in Wasser-in-Öl-Emulsionen umwandeln. Beim anschließenden Abkühlen werden wieder O/W-Emulsionen gebildet, die aber auch bei Raumtemperatur als Mikroemulsionen oder als sehr feinteilige Emulsionen mit einem mittleren Teilchendurchmesser unter 400 nm und insbesondere von etwa 100-300 nm, vorliegen. Erfindungsgemäß können solche Mikro- oder "PIT"-Emulsionen bevorzugt sein, die einen mittleren Teilchendurchmesser von etwa 200 nm aufweisen.

In der bevorzugten Ausführungsform als Emulsion enthalten die erfindungsgemäßen Zusammensetzungen mindestens eine oberflächenaktive Substanz als Emulgator oder Dispergiermittel. Geeignete Emulgatoren sind beispielsweise Anlagerungsprodukte von 4 bis 30 Mol Ethylenoxid und/oder 0 bis 5 Mol Propylenoxid an lineare $C_8$-$C_{22}$-Fettalkohole, an $C_{12}$-$C_{22}$-Fettsäuren und an $C_8$-$C_{15}$-Alkylphenole, $C_{12}$-$C_{22}$-Fettsäuremono- und -diester von

Anlagerungsprodukten von 1 bis 30 Mol Ethylenoxid an $C_3$-$C_6$-Polyole, insbesondere an Glycerin, Ethylenoxid- und Polyglycerin-Anlagerungsprodukte an Methylglucosid-Fettsäureester, Fettsäurealkanolamide und Fettsäureglucamide, $C_8$-$C_{22}$-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind, Gemische aus Alkyl-(oligo)-glucosiden und Fettalkoholen, z. B. das im Handel erhältliche Produkt Montanov® 68, Anlagerungsprodukte von 5 bis 60 Mol Ethylenoxid an Rizinusöl und gehärtetes Rizinusöl, Partialester von Polyolen mit 3-6 Kohlenstoffatomen mit gesättigten $C_8$-$C_{22}$-Fettsäuren, Sterole (Sterine), insbesondere Cholesterol, Lanosterol, Beta-Sitosterol, Stigmasterol, Campesterol und Ergosterol sowie Mykosterole, Phospholipide, vor allem Glucose-Phospolipide, Fettsäureester von Zuckern und Zuckeralkoholen wie Sorbit, Polyglycerine und Polyglycerinderivate, bevorzugt Polyglyceryl-2-dipolyhydroxystearat (Handelsprodukt Dehymuls® PGPH) und Polyglyceryl-3-diisostearat (Handelsprodukt Lameform® TGI) sowie lineare und verzweigte $C_8$-$C_{30}$-Fettsäuren und deren Na-, K-, Ammonium-, Ca-, Mg- und Zn-Salze.

Die erfindungsgemäßen Zusammensetzungen enthalten die Emulgatoren bevorzugt in einer Gesamtmenge von 0,1 bis 25 Gew.-%, besonders bevorzugt 0,5 - 15 Gew.-%, bezogen auf die gesamte Zusammensetzung.

In einer besonders bevorzugten Ausführungsform ist mindestens ein nichtionischer Emulgator mit einem HLB-Wert von 8 und darunter enthalten. Derartige bevorzugte Emulgatoren sind insbesondere Verbindungen der allgemeinen Formel $R^1$ - O - $R^2$, in der $R^1$ eine primäre lineare Alkyl-, Alkenyl- oder Acylgruppe mit 20 - 30 C-Atomen, bevorzugt ein Behenyl-Rest, und $R^2$ Wasserstoff, eine Gruppe mit der Formel $-(C_nH_{2n}O)_x$-H mit x = 1 oder 2 und n = 2 - 4 oder eine Polyhydroxyalkylgruppe mit 4 - 6 C-Atomen und 2 - 5 Hydroxylgruppen, bevorzugt Wasserstoff, darstellt. Eine besonders bevorzugte Verbindung $R^1$ - O - $R^2$ ist Behenylalkohol. Weitere bevorzugt geeignete Emulgatoren mit einem HLB-Wert von 8 und darunter sind die Anlagerungsprodukte von 1 oder 2 Mol Ethylenoxid oder Propylenoxid an Behenylalkohol, Erucylalkohol, Arachidylalkohol oder auch an Behensäure oder Erucasäure. Bevorzugt eignen sich auch die Monoester von $C_{16}$-$C_{30}$-Fettsäuren mit Polyolen wie z. B. Pentaerythrit, Trimethylolpropan, Diglycerin, Sorbit, Glucose oder Methylglucose. Beispiele für solche Produkte sind z. B. Sorbitanmonobehenat oder Pentaerythritmonoerucat. Die Anwesenheit von Emulgatoren der allgemeinen Formel $R^1$ - O - $R^2$ kann zu lamellaren Strukturen in der Emulsion führen, die besonders günstige Effekte auf die Wiederherstellung der lamellaren Struktur der Haut haben können. Besonders bevorzugte erfindungsgemäße Zusammensetzungen sind daher **dadurch gekennzeichnet, dass** sie in Form einer lamellare Strukturen aufweisenden Öl-in-Wasser-Emulsion vorliegen.

In einer weiteren besonders bevorzugten Ausführungsform liegen die erfindungsgemäßen Zusammensetzungen in Form einer Wasser-in-Öl-Emulsion vor. Derartige Wasser-in-Öl-Emulsionen können so formuliert werden, dass die eine höhere Viskosität aufweisen, so dass sie sorgfältig in die Haut einzumassieren sind. Dies unterstützt die Anticellulite-Wirkung der erfindungsgemäßen Zusammensetzungen. Genau so gut lassen sich aber auch niedrig-viskose Wasser-in-Öl-Emulsionen formulieren, insbesondere auf Basis des polymeren Wasser-in-Öl-Emulgators PEG-30 Dipolyhydroxystearat, erhältlich z. B. unter dem Handelsnamen Arlacel P 135 von Uniqema. Insbesondere mit Hilfe dieses Emulgators lassen sich sprühbare Wasser-in-Öl-Emulsionen formulieren.

Weitere geeignete Zusatzstoffe sind Verdickungsmittel, z. B. anionische Polymere aus Acrylsäure, Methacrylsäure, Crotonsäure, Maleinsäureanhydrid und 2-Acrylamido-2-methylpropansulfonsäure, wobei die sauren Gruppen ganz oder teilweise als Natrium-, Kalium-, Ammonium-, Mono- oder Triethanolammonium-Salz vorliegen können und wobei mindestens ein nichtionisches Monomer enthalten sein kann. Bevorzugte nichtionogene Monomere sind Acrylamid, Methacrylamid, Acrylsäureester, Methacrylsäureester, Vinylpyrrolidon, Vinylether und Vinylester. Bevorzugte anionische Copolymere sind Acrylsäure-Acrylamid-Copolymere sowie insbesondere Polyacrylamidcopolymere mit Sulfonsäuregruppen-haltigen Monomeren. Diese Copolymere können auch vernetzt vorliegen. Geeignete Handelsprodukte sind Sepigel®305, Simulgel® 600, Simulgel® NS und Simulgel® EG der Firma SEPPIC. Weitere besonders bevorzugte anionische Homo- und Copolymere sind unvernetzte und vernetzte Polyacrylsäuren. Solche Verbindungen sind zum Beispiel die Handelsprodukte Carbopol®. Ein besonders bevorzugtes anionisches Copolymer enthält als Monomer zu 80 - 98 % eine ungesättigte, gewünschtenfalls substituierte $C_{3-6}$-Carbonsäure oder ihr Anhydrid sowie zu 2 - 20 % gewünschtenfalls substituierte Acrylsäureester von gesättigten $C_{10-30}$-Carbonsäuren, wobei das Copolymer mit den vorgenannten Vernetzungsagentien vernetzt sein kann. Entsprechende Handelsprodukte sind Pemulen® und die Carbopol®-Typen 954, 980, 1342 und ETD 2020 (ex B.F. Goodrich).

Geeignete nichtionische Polymere sind beispielsweise Polyvinylalkohole, die teilverseift sein können, z. B. die Handelsprodukte Mowiol® sowie Vinylpyrrolidon/Vinylester-Copolymere und Polyvinylpyrrolidone, die z. B. unter dem Warenzeichen Luviskol® (BASF) vertrieben werden.

Weitere bevorzugte Zusatzstoffe sind Lösungsmittel wie Ethanol, Isopropanol, Ethylenglykol, Propylenglykol, Propylenglykolmonoethylether, Glycerin und Diethylenglykol, Parfümöle, Substanzen zur Einstellung des pH-Wertes, Komplexbildner wie EDTA, NTA, β-Alanindiessigsäure und Phosphonsäuren, Treibmittel wie Propan-Butan-Gemische, Pentan, Isopentan, Isobutan, $N_2O$, Dimethylether, $CO_2$ und Luft.

In einer weiteren bevorzugten Ausführungsform liegen die erfindungsgemäßen Zusammensetzungen in einem Sprühspender oder Pumpspender verpackt vor.

Die nachfolgenden Beispiele sollen den Gegenstand der vorliegenden Anmeldung verdeutlichen, ohne ihn

hierauf einzuschränken.

Beispiele:

Die Zahlenwerte in den Beispielen bedeuten Gewichtsprozente, bezogen auf das Gesamtgewicht der jeweiligen Zusammensetzungen.

Die Methode der Antioxidative Power (AP) ermöglicht die Bestimmung der antioxidativen Aktivität von aktiven Inhaltsstoffen in kosmetischen Produkten wie z.B. Pflanzenextrakten und Vitaminen. Die AP basiert auf der ESR (Elektronen Spin Resonanz) Spektroskopie, einer Methode, mit der freie Radikale quantitativ und qualitativ charakterisiert werden können. Damit impliziert die Methode auch die quantitative und qualitative Charakterisierung von Antioxidantien und Radikalfängern als den Antagonisten der freien Radikale.

Die Antioxidative Power ist eine zweidimensionale Größe, die sowohl die Kapazität eines Antioxidans, freie Radikale zu neutralisieren angibt, als auch seine Reaktivität. Die Kapazität wird anhand einer konzentrationsabhängigen Signalintensität ermittelt; die Reaktivität wird über das Erstellen von Reaktionskinetiken gemessen. So werden sowohl die Reaktionszeit ($t_r$) als auch die radikalreduzierende Kapazität erfasst und beide Größen fließen in die Berechnung der AP ein. Je schneller eine Substanz mit den freien Radikalen reagiert, desto niedriger ist ihre Reaktionszeit ($t_r$), desto höher ist ihre Antioxidative Power. Die Reaktionszeit ($t_r$) ist ein wichtiges Indiz für die Wirksamkeit einer Substanz und ihres Oxidationsgrades und ist ein Identifikationsmerkmal für ein Antioxidans.

Die AP gibt an, wie viele Radikale von 1 mg Substanz in 1 Minute neutralisiert werden und wird in (rad/m*min) angegeben. Die AP Methode ist auf die Aktivität von Vitamin C (Ascorbinsäure) als Bezugspunkt standardisiert worden, so dass die AP in Antioxidative Units (AU) angegeben werden kann, wobei 1 AU der Aktivität von 1 ppm Vitamin C entspricht. Ein AP einer Substanz von 100 AU bedeutet, dass 1 mg der Substanz die gleiche Menge Radikale pro Zeiteinheit neutralisieren kann wie 0,1 pg Vitamin C.

Radical Skin Protection Factor (RSF)

Bei der RSF Methode werden die durch UV-Strahlung induzierten freien Radikale in der Haut gemessen. An Hand von experimentell bestimmten Gleichungen und mathematisch durchgeführten Kalibrierungen kann der Schutzfaktor (RSF), verursacht durch die topisch applizierten Produkte, bestimmt werden. Dabei gilt für die RSF-Bestimmung folgender Zusammenhang:

$$\text{RSF} = \text{Radikalmenge in ungeschützter Haut/Radikalmenge in geschützter Haut.}$$

Ähnlich dem SPF wird bei der Bestimmung des RSF die Wirkung der UV-Strahlung auf der Haut gemessen. Als Messgröße bzw. Indikator im Vergleich zwischen unbestrahlter und bestrahlter Haut wird nicht das als biologischer Endpunkte bezeichnete Erythem verwendet, sondern es wird die Menge und Art an UV generierten freien Radikalen in der Haut bestimmt. Die Menge an freien Radikalen ist proportional zur UV-Dosis und ihre Verteilung in den einzelnen Hautschichten (Epidermis, Dermis) wird maßgeblich von der Wellenlänge der eindringenden Photonen bestimmt. Die Bestimmung des RSF wird zweckmäßigerweise als ex vivo Methode an Hautbiopsien durchgeführt. Die Hautbiopsien werden mit einem Radikalindikator (Nitroxyl-Verbindung) markiert. Die durch die UV-Strahlung in der Haut generierten freien Radikale reagieren mit dem Radikalindikator, dessen Menge und Aktivität mit einem ESR-Spektrometer gemessen wird. Die Bildung der durch UVA und UVB induzierten freien Radikale ist ein Prozess, der sich in der epidermalen und dermalen Schicht der Haut abspielt. Die Wirkung der UVB-Strahlung beschränkt sich, bedingt durch die geringe Eindringtiefe, hauptsächlich auf die Epidermis. Der Radikalindikator ist hydrophil und weist demzufolge in der Dermis und Epidermis eine morphologisch bedingte Gleichverteilung auf. Der Radikalindikator selbst ist fotostabil und wird in wässriger Lösung nicht durch UV abgebaut.

Die in der Haut induzierten freien Radikale oxidieren das Molekül der Nitroxylverbindung und werden mit der ESR-Spektroskopie nachgewiesen. Über diesen Effekt kann die UV-schützende Wirkung (Radikalschutz) von UV-Filtern getestet werden. Während das UVA ausschließlich freie Radikale in der Haut generiert, kann das UVB (insbesondere das kurzwellige UVB) auch zu direkten Schäden an der Erbinformation (DANN) der Zelle führen. Dementsprechend ist die RSF-Methode besonders sensitiv gegenüber der Wirkung von UVA-Filtern.

Gleiche UV-Dosis für beide Messungen vorausgesetzt. Es wird ein Faktor angegeben, um den die generierte Radikalmenge bei Benutzung eines Sonnenschutzproduktes verringert wird.

Ebenfalls kann für eine gleiche vorgegebene induzierte Radikalmenge in beiden Fällen (ungeschützt versus geschützt) der Faktor angegeben werden, um den die Verweildauer unter UV-Strahlung für geschützte Haut erhöht

werden kann. Eine direkte Bezugnahme zum SPF ist damit gegeben.

Hautmodell

Das verwendete Hautmodell basiert auf der Verwendung von Schweinehaut von Schlachttieren.

Das Schweinehautmodell ist als dasjenige anerkannt, das der menschlichen Haut am ähnlichsten ist. Die Schweinehaut wurde unmittelbar nach der Schlachtung (innerhalb von 4 Stunden) für die Hautuntersuchungen präpariert. Bis zur Messung wurden die Schweinehautstreifen (>1 cm x 1 cm) auf einer PBS-Lösung getränkten Gaze im Kühlschrank gelagert. Das Alter der Schlachttiere liegt einheitlich bei ca. 6 Monaten, so dass große altersbedingte Hautunterschiede nicht zu erwarten sind und bisher nicht beobachtet wurden.

Die Durchführung des Testverfahrens ist im Wesentlichen gekennzeichnet durch zwei Prozesse:

1. Einwirkung des Radikalindikators von der dermalen (5 min.) und der Probe der epidermalen (15 min.) Seite der Haut.
2. Die UV-Bestrahlung der Haut mit variablen Zeiten (entsprechend Versuchsprotokoll) von der epidermalen Seite und anschließender unmittelbarer Messung der induzierten freien Radikale mit der ESR-Spektroskopie.

Aus dem 1 $cm^2$ Hautareal wird eine 4 mm ∅ große Biopsie ausgestanzt und auf einer ESR-Gewebezelle montiert. Danach erfolgt eine erste ESR-Messung (0-Messung). Die Biopsie wird mit zunehmenden UV-Dosen (UV-Bestrahlungszeiten) bestrahlt. Nach jeder Messung erfolgt eine Messung der Radikalintensität.

Die Bestrahlung erfolgt mit einem Sonnensimulator der Firma Hönle AG, Modell SOL2, bei einer Bestrahlungsstärke von E(UVA + UVB) = 23,2 mW/$cm^2$. Die einzelnen Bestrahlungsintensitäten betragen 22,0 mW/$cm^2$ für UVA und 1,2 mW/$cm^2$ für UVB. Das E(UVA)/E(UVB)-Verhältnis ist 22/2,1 = 18,3. Dieses Verhältnis entspricht der COLIPA-Anforderung, die sich nach den Sonnenintensitäts-Verhältnissen für die einzelnen Spektralbereiche richtet.

Es wurden folgende Rezepturen hergestellt.

| Beschreibung | A: Placebo | B: Lipochroman | C: UV-Filter | D: Lipochroman +UV-Filter |
|---|---|---|---|---|
| Emulsiphos 677660 | 3 | 3 | 3 | 3 |
| Cetiol B | 5 | 5 | 5 | 5 |
| Estasan GT8-60 3575 MCT Oil | 3,5 | 3,5 | 3,5 | 3,5 |
| Safloröl raffiniert | 2 | 2 | 2 | 2 |
| Behenylalkohol | 3 | 3 | 3 | 3 |
| Cetiol Sensoft | 1 | 1 | 1 | 1 |
| Vitamin E Acetat | 0,5 | 0,5 | 0,5 | 0,5 |
| Controx KS | 0,05 | 0,05 | 0,05 | 0,05 |
| Bienenwachs | 0,2 | 0,2 | 0,2 | 0,2 |
| COSMEDIA SP | 0,5 | 0,5 | 0,5 | 0,5 |
| Ethylparaben | 0,2 | 0,2 | 0,2 | 0,2 |
| Wasser, vollentsalzt | 65,4 | 65,39 | 54,975 | 54,965 |
| Hexandiol-1,6 | 6 | 6 | 6 | 6 |
| Glycerin 86% pflanzlich | 4,5 | 4,5 | 4,5 | 4,5 |
| Sorbit 70% LS DAB | 2 | 2 | 2 | 2 |
| Methylparaben | 0,2 | 0,2 | 0,2 | 0,2 |

| | | | | |
|---|---|---|---|---|
| Tego Carbomer 140 | 0,5 | 0,5 | 0,5 | 0,5 |
| EDETA BX Powder | 0,05 | 0,05 | 0,05 | 0,05 |
| Phenoxyethanol, rein | 0,4 | 0,4 | 0,4 | 0,4 |
| Cosmedia Silc | 2 | 2 | 2 | 2 |
| Lipochroman-6 | | 0,01 | | 0,01 |
| Butylmethoxydibenzoylmethan | | | 3 | 3 |
| Parsol SLX | | | 3 | 3 |
| Natriumhydroxid Perlen | | | 0,435 | 0,435 |
| AMP Ultra PC 1000 | | | 0,99 | 0,99 |
| Neo Heliopan Hydro | | | 3 | 3 |

Die Beispiele A, B und C sind nicht erfindungsgemäß, Beispiel 4 ist erfindungsgemäß.

Es wurden mittels der zuvor beschriebenen Methode folgende AU-Werte ermittelt:

| | |
|---|---|
| A | 100 AU |
| B | 245 AU |
| C | 99 AU |
| D | 396 AU |

Hieraus resultieren folgende RSF-Werte:

| | |
|---|---|
| A | 1,0 +/- 0,2 |
| B | 1,1 +/- 0,2 |
| C | 5,7 +/- 0,3 |
| D | 13,6 +/- 0,8 |

Die Menge UV-induzierter radikale, die reduziert wurde ist damit:

| | |
|---|---|
| A | 0 % |
| B | 0 & |
| C | 82 % |
| D | 99 % |

## Patentansprüche

**1.** Kosmetische Zusammensetzung, enthaltend

a) mindestens einen öllöslichen UV-Filter, umfassend mindestens ein Alkyl- und/oder Alkoxy-substituiertes Dibenzoylmethanderivat,
b) Polysilicone-15,
c) mindestens einen wasserlöslichen UV-Filter, umfassend ein Derivat der Benzimidazolsulfonsäure,
d) mindestens eine Substanz, ausgewählt aus 6,7-disubstituierten 2,2-Dialkylchromanen oder -chromenen der

allgemeinen Formeln (II) oder (III),

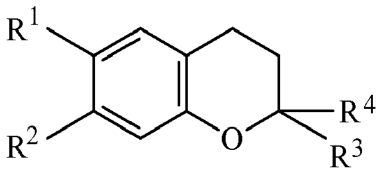

(I)

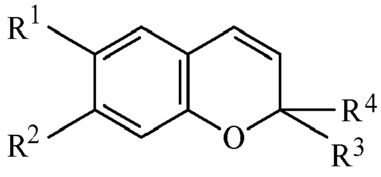

(II)

wobei $R^1$ und $R^2$ unabhängig voneinander eine OH-Gruppe, eine Methoxy-Gruppe oder eine $CF_3CH_2O$-Gruppe und $R^3$ und $R^4$ unabhängig voneinander eine $C_1$-$C_4$-Alkylgruppe darstellen, insbesondere Lipochroman- 6,

wobei folgende Zusammensetzungen vom Schutzbereich ausgenommen sind:

| | | | | | | | | |
|---|---|---|---|---|---|---|---|---|
| Rizinusöl | - | - | - | - | - | - | - | 3,0 |
| C16-36 Alkyl Stearate | - | - | - | - | 1,0 | 2,0 | 1,0 | - |
| C20-40 Alkyl Stearate | - | - | - | - | 8,0 | 8, 0 | 9,0 | 8,0 |
| PEG-30 Stearat | 1,5 | - | - | - | - | - | - | - |
| PEG-40 Stearat | - | 2,0 | - | - | - | - | - | - |
| PEG-100 Stearat | - | - | - | 1,0 | - | - | - | - |
| Glyceryl Stearat | 0,5 | 1, 2 | - | 3,0 | - | - | - | - |
| Ceteareth-20 | 1,0 | - | - | - | - | - | - | - |
| Stearinsäure | 2,0 | - | - | - | - | - | - | - |
| Glyceryl Stearat Citrat | - | - | 3,5 | - | - | - | - | - |
| Cetyl Alkohol | 0,5 | 2,0 | 0,75 | 1,0 | - | - | - | - |
| Cetylpalmitat | - | 1,0 | - | - | - | - | - | 1,0 |
| Cetearylalkohol | - | - | - | - | - | - | 1,0 | - |
| Carnaubawachs | - | - | - | - | 1,5 | 0,5 | 2,0 | 2,0 |
| Veegum K = Mg-Al-Silikat | 0,8 | - | - | - | - | - | - | - |
| Xanthan Gummi | 0,2 | - | - | - | - | - | - | - |
| Carbomer | - | 0,3 | 0,2 | 0,2 | - | - | - | - |
| Hydroxyethylcellulose | 0,2 | - | - | - | - | - | - | - |
| Capryl-/Caprinsäure | - | - | 2,0 | - | 5,0 | 5,0 | 8,0 | 3,0 |
| Triglycerid | | | | | | | | |
| Pentaerythrityl Tetraisostearat | - | - | - | - | 4,0 | - | 8,0 | - |
| Caprylylcarbonat | - | - | - | - | - | 5,0 | - | - |
| Jojobaöl | - | - | - | - | 1,0 | 1,0 | 1,0 | 1,0 |
| Lanolinöl | - | - | - | - | - | - | - | 1,0 |
| PEG-45/ Dodecyl Glycol Copolymer | - | - | - | - | 3,5 | 2,0 | 2,0 | 2,0 |
| Polyglyceryl-3 Diisostearat | - | - | - | - | - | 1,5 | 2,0 | 2,4 |
| Bis-Diglyceryl Polyacyladipat-2 | - | - | - | - | 2,0 | - | - | - |
| PVP / Eicosene Copolymer | - | - | - | - | - | - | 1,0 | 0,2 |
| Octyldodecanol | - | - | - | - | 5,0 | 5,0 | 8,0 | 6,0 |

(fortgesetzt)

| | | | | | | | | |
|---|---|---|---|---|---|---|---|---|
| Dicaprylylether | - | 3,0 | 2,0 | - | - | - | - | - |
| Dimethicone | 3,0 | 2,0 | - | - | - | - | - | - |
| Simethicone | - | - | - | - | 0,5 | 0,5 | - | 0,5 |
| Cyclomethicone | - | - | 4,0 | 1,0 | - | - | - | - |
| C12-C15 Alkyl Benzoat | 2,0 | - | - | - | - | - | - | - |
| Cetearyloctanoat | 2,0 | - | - | - | - | - | - | - |
| Squalan | 1,0 | - | - | 1,0 | - | - | - | - |
| Isopropylpalmitat | 1,0 | - | - | - | - | - | - | 2,0 |
| PPG-15 Stearylether | 2,0 | 2, 0 | 3,0 | - | - | - | - | - |
| Vaseline | - | - | - | 2,0 | - | - | - | - |
| Hydrierte Kokosglyceride | 2,0 | - | - | 1,0 | - | - | - | - |
| Hydrierte Polydecen | | 2,0 | | | - | - | - | - |
| Stearyl Dimethicone | 9,0 | - | - | - | - | - | - | - |
| Ethylhexyl Methoxycinnamat | - | 4,50 | - | - | - | - | 1,0 | - |
| Bis-Ethylhexyloxyphenol methoxyphenyl Triazine | 1,00 | 2,50 | 3,00 | - | 0,50 | 1,50 | 3,50 | - |
| Butyl Methoxy Dibenzoylmethane | 2,00 | 2,50 | 3,00 | 2,00 | 3,0 | 3,0 | 3,0 | 2,00 |
| Neo Heliopan AP | - | - | - | 2,00 | - | - | - | 0,75 |
| Ethylhexyl Triazone | - | 1,50 | - | - | 3,50 | - | - | - |
| Octocrylene | 3,00 | - | - | - | - | - | - | - |
| Diethylhexyl Butamido Triazone | - | - | 3,00 | - | - | 2,00 | 0,75 | |
| Neo Heliopan Hydro | 2,00 | 3,00 | 3,00 | - | - | - | - | - |
| Methylen Bis-Benzotriazolyl Tetramethylbutylphenol | 2,00 | 1,50 | - | - | - | - | - | 2,50 |
| Eusolex T-AVO | - | - | - | - | 2,0 | 1,0 | 4,0 | - |
| 2-Phenylbenzimidazol-5-sulfonsäure | - | - | - | - | 3,0 | 3,0 | 3,0 | 1,00 |
| Ethylhexyl Salicylate | - | - | - | - | - | 3,50 | - | - |
| Homosalate | - | - | - | - | 2,00 | - | - | - |
| Polysilicone-15 | 3,00 | 3,00 | 2,00 | 2,00 | 3,0 | 3,0 | 3,0 | 2,00 |
| Diethylhexyl-2,6-naphthalat | - | - | - | 3,50 | - | - | - | 5,50 |
| Ronasphere LDP | 1,0 | - | 4,0 | - | - | - | - | - |
| Tospearl 145A | - | 5, 0 | - | 3,0 | 3,0 | 0,5 | 1,0 | 5,0 |
| $BiOCl_3$ | - | - | - | - | - | 3,0 | - | - |
| Bornitrid | - | - | - | - | - | - | 3,0 | 1,0 |
| Lauroyl Lysin | - | - | - | - | 0,5 | - | - | - |
| Micropearl® M 310 | - | - | - | - | 6,0 | 3,0 | - | - |
| Siliciumdioxid-Partikel* | 2,00 | 1,50 | 2,00 | 1,50 | 2,00 | 2,00 | 2,00 | 2,00 |
| Eisenoxide CI 77491, 77492, 77499 | 1,2 | - | 0,8 | 2,6 | 3,6 | 1,8 | 0,8 | 3,2 |

(fortgesetzt)

| | | | | | | | | |
|---|---|---|---|---|---|---|---|---|
| Titandioxid, CI 77891 | 3,8 | - | 1,2 | 4,5 | 5,0 | 3, 6 | 1,2 | 4,5 |
| Ultramarin, CI 77007 | 0,5 | - | - | 0,6 | - | - | - | - |
| Interferenzpigmente | 0,8 | 6, 0 | - | - | - | - | 1,0 | - |
| D&C Rot 7 | - | - | - | - | - | - | - | 4,5 |
| D&C Rot 6 | - | - | - | - | - | - | - | 0,2 |
| FD&C Blau 1 | - | - | - | - | - | - | - | 0,5 |
| D&C Rot 21 | - | - | - | - | 0,1 | - | - | - |
| FD&C Gelb 6 | - | - | - | - | - | - | - | 0,2 |
| D&C Rot 34 | - | - | - | - | - | - | - | 0,5 |
| Glycerin | 2,0 | 2,0 | 5,0 | 10,0 | - | 10 | - | 10 |
| Citronensäure | - | - | - | - | - | 0,05 | - | - |
| Panthenol | - | - | - | - | - | - | 1,0 | - |
| Parfum | 1,0 | 1,0 | 1,0 | 1,0 | 1,0 | 1,0 | 1,0 | 1,0 |
| Methylparaben | 1,0 | 1,0 | 1,0 | 1,0 | 1,0 | 1,0 | 1,0 | 1,0 |
| Propylparaben | 1,0 | 1,0 | 1,0 | 1,0 | 1,0 | 1,0 | 1,0 | 1,0 |
| Lipochroman-6 | 0,5 | 0,5 | 0,5 | 0,5 | 0,5 | 0,5 | 0,5 | 0,5 |
| Wasser | ad 100 | ad 100 | ad 100 | ad 100 | ad 100 | ad 100 | ad 100 | ad 100 |

* nicht organisch oder anorganisch modifiziert, keine organische oder anorganische Beschichtung, zu mehr als 80 Gew.-% sphärische Partikel, zahlenmittlerer Partikeldurchmesser 6 - 8 μm, Ölabsorptionskapazität : 0,9 - 1,3 $cm^3$ flüssiges Paraffin pro Gramm trockenes Siliciumdioxid (DIN 53601), erhalten durch Kieselsäure-Fällung

und wobei folgende Zusammensetzungen ebenfalls vom Schutzbereich ausgenommen sind:

| | | | | |
|---|---|---|---|---|
| Emulsiphos 677660 | 3 | 3 | 3 | 3 |
| Cetiol B | 5 | 5 | 5 | 5 |
| Estasan GT8-60 3575 MCT Oil | 3,5 | 3,5 | 3,5 | 3,5 |
| Safloröl raffiniert | 2 | 2 | 2 | 2 |
| Behenylalkohol | 3 | 3 | 3 | 3 |
| Cetiol Sensoft | 1 | 1 | 1 | 1 |
| Vitamin E Acetat | 0,5 | 0,5 | 0,5 | 0,5 |
| Controx KS | 0,05 | 0,05 | 0,05 | 0,05 |
| Bienenwachs | 0,2 | 0,2 | 0,2 | 0,2 |
| COSMEDIA SP | 0,5 | 0,5 | 0,5 | 0,5 |
| Butylmethoxydibenzoylmethan | 3 | 3 | 3 | 3 |
| Parsol SLX | 3 | 3 | 3 | 3 |
| Propylparaben | 0,2 | 0,2 | 0,2 | 0,2 |
| Hexandiol-1,6 | 5 | 5 | 5 | 5 |
| Glycerin 86% pflanzlich | 4,5 | 4,5 | 4,5 | 4,5 |
| Sorbit 70% LS DAB | 2 | 2 | 2 | 2 |

(fortgesetzt)

| | | | | |
|---|---|---|---|---|
| Methylparaben | 0,2 | 0,2 | 0,2 | 0,2 |
| Tego Carbomer 140 | 0,5 | 0,5 | 0,5 | 0,5 |
| NTA-Na3 flüssig 40% | 0,1 | 0,1 | 0,1 | 0,1 |
| Cosmedia Silc | 2 | 2 | 2 | 2 |
| AMP Ultra PC 1000 | 0,99 | 0,99 | 0,99 | 0,99 |
| Neo Heliopan Hydro | 3 | 3 | 3 | 3 |
| Natriumhydroxid Perlen | 0,365 | 0,365 | 0,365 | 0,365 |
| Epicalmin TCM | - | - | 3 | 2 |
| Phycojuvenine | 2 | 1 | 2 | 1 |
| Lipochroman | 0,01 | 0,05 | 0,01 | 0,05 |
| Wasser, vollentsalzt | ad 100 | ad 100 | ad 100 | ad 100 |

**2.** Kosmetische Zusammensetzung gemäß Anspruch 1, **dadurch gekennzeichnet, dass** sie - bezogen auf ihr Gewicht - 0,0001 bis 10 Gew.-%, vorzugsweise 0,0005 bis 7,5 Gew.-%, weiter bevorzugt 0,001 bis 5 Gew.-%, noch weiter bevorzugt 0,002 bis 2,5 Gew.-% und insbesondere 0,005 bis 2 Gew.-% 6,7-disubstituierte 2,2-Dialkylchromane oder -chromene der allgemeinen Formeln (II) oder (III) wie vorstehend definiert, enthält, wobei bevorzugte Zusammensetzungen - bezogen auf ihr Gewicht - 0,0001 bis 10 Gew.-%, vorzugsweise 0,0005 bis 7,5 Gew.-%, weiter bevorzugt 0,001 bis 5 Gew.-%, noch weiter bevorzugt 0,002 bis 2,5 Gew.-% und insbesondere 0,005 bis 2 Gew.-% Lipochroman-6 enthalten.

**3.** Kosmetische Zusammensetzung gemäß Anspruch 1 oder 2, **dadurch gekennzeichnet, dass** der mindestens eine UV-Filter a) ausgewählt ist aus 4-(tert.-Butyl)-4'-methoxydibenzoylmethan.

**4.** Kosmetische Zusammensetzung gemäß Anspruch 1, 2 oder 3, **dadurch gekennzeichnet, dass** der mindestens eine UV-Filter c) ausgewählt ist aus Phenylen-1,4-bis-(2-benzimidazyl)-3,3'-5,5'-tetrasulfonsäure, 2-Phenylbenzimidazol-5-sulfonsäure und den Salzen dieser Säuren, bevorzugt den entsprechenden Natrium-, Kalium- oder Triethanolammonium-Salzen, insbesondere aus Phenylbenzimidazolsulfonsäure und dem Phenylen-1,4-bis-(2-benzimidazyl)-3,3'-5,5'-tetrasulfonsäure-bis-natriumsalz.

**5.** Kosmetische Zusammensetzung gemäß Anspruch 1, 2, 3 oder 4, **dadurch gekennzeichnet, dass** der mindestens eine UV-Filter c) ausgewählt ist aus den Salzen der 2-Phenylbenzimidazol-5-sulfonsäure mit basischen Aminosäuren, insbesondere mit Lysin, Arginin und/oder Histidin, wobei Arginin besonders bevorzugt ist.

**6.** Kosmetische Zusammensetzung gemäß Anspruch 1, 2, 3, 4 oder 5, **dadurch gekennzeichnet, dass** der mindestens eine UV-Filter c) ausgewählt ist aus den Salzen der Phenylen-1,4-bis-(2-benzimidazyl)-3,3'-5,5'-tetrasulfonsäure mit basischen Aminosäuren, insbesondere mit Lysin, Arginin und/oder Histidin, wobei Arginin besonders bevorzugt ist.

**7.** Kosmetische Zusammensetzung gemäß Anspruch 1, 2, 3, 4, 5 oder 6, **dadurch gekennzeichnet, dass** 4-(tert.-Butyl)-4'-methoxydibenzoylmethan, Polysilicone-15 und 2-Phenylbenzimidazol-5-sulfonsäure enthalten sind.

**8.** Kosmetische Zusammensetzung gemäß Anspruch 1, 2, 3, 4, 5, 6 oder 7, **dadurch gekennzeichnet, dass** 4-(tert.-Butyl)-4'-methoxydibenzoylmethan, Polysilicone-15 und 2-Phenylbenzimidazol-5-sulfonsäure enthalten sind.

**9.** Kosmetische Zusammensetzung gemäß Anspruch 1, 2, 3, 4, 5, 6, 7 oder 8, **dadurch gekennzeichnet, dass** die UV-Filter a), b) und c) in einem Gewichtsverhältnis zueinander von a) : b) : c) wie (0,8 - 1,5) : (0,8 - 1,5) : (0,8 - 1,5) enthalten sind.

**10.** Kosmetische Zusammensetzung gemäß Anspruch 1, 2, 3, 4, 5, 6, 7, 8 oder 9, **dadurch gekennzeichnet, dass** die UV-Filter a), b) und c) in einem Gewichtsverhältnis zueinander von a): b) : c) wie (0,9 - 1,2) : (0,9 - 1,2) : (0,9-

1,2), bevorzugt wie (1 - 1,1): (1 - 1,1) : (1 - 1,1), enthalten sind.

**11.** Kosmetische Zusammensetzung gemäß Anspruch 1, 2, 3, 4, 5, 6, 7, 8, 9 oder 10, **dadurch gekennzeichnet, dass** sie zu 0,2 - 5 Gew.-% Siliciumdioxid-Partikel, die nicht organisch oder anorganisch modifiziert sind und keine organische oder anorganische Beschichtung aufweisen, enthalten sind.

**12.** Kosmetische Zusammensetzung gemäß Anspruch 11, **dadurch gekennzeichnet, dass** die Siliciumdioxid-Partikel zu mehr als 80 Gew.-% sphärische Partikel umfassen.

**13.** Kosmetische Zusammensetzung gemäß Anspruch 11oder 12, **dadurch gekennzeichnet, dass** die Siliciumdioxid-Partikel einen zahlenmittleren Partikeldurchmesser im Bereich von 2 - 15 μm aufweisen.

**14.** Kosmetische Zusammensetzung gemäß Anspruch 11, 12 oder 13, **dadurch gekennzeichnet, dass** die Siliciumdioxid-Partikel einen zahlenmittleren Partikeldurchmesser im Bereich von 5 - 10 μm aufweisen.

**15.** Kosmetische Zusammensetzung gemäß Anspruch 11, 12, 13 oder 14, **dadurch gekennzeichnet, dass** die Siliciumdioxid-Partikel eine Ölabsorptionskapazität von 0,7 - 1,5 $cm^3$ flüssiges Paraffin pro Gramm trockenes Siliciumdioxid, aufweisen, gemessen nach DIN 53601.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- DE 102008025576
- EP 709080 A2
- EP 775698 A
- EP 878469 A
- EP 1027881 A
- DE 102009017612
- DE 102009